(19) Europäisches Patentamt
European Patent Office
Office européen des brevets

(11) EP 4 321 511 A1

(12) **EUROPEAN PATENT APPLICATION**

(43) Date of publication:
**14.02.2024 Bulletin 2024/07**

(21) Application number: **22189496.7**

(22) Date of filing: **09.08.2022**

(51) International Patent Classification (IPC):
***C07D 401/10*** *(2006.01)* ***C09K 11/06*** *(2006.01)*

(52) Cooperative Patent Classification (CPC):
**C07D 401/10; C09K 11/06;** C09K 2211/1425; C09K 2211/1433; C09K 2211/1458; C09K 2211/1466

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Novaled GmbH**
**01307 Dresden (DE)**

(72) Inventors:
- **GALÁN GARCÍA, Elena**
**01099 Dresden (DE)**
- **KARPOV, Yevhen**
**01099 Dresden (DE)**

(74) Representative: **Bittner, Thomas L.**
**Boehmert & Boehmert**
**Anwaltspartnerschaft mbB**
**Pettenkoferstrasse 22**
**80336 München (DE)**

(54) **COMPOUND, ORGANIC SEMICONDUCTING MATERIAL COMPRISING THE SAME, ORGANIC ELECTRONIC DEVICE COMPRISING THE SAME AND DISPLAY DEVICE COMPRISING THE SAME**

(57) The present invention relates to a compound, an organic semiconducting material comprising the same, an organic electronic device comprising the same and a display device comprising the same.

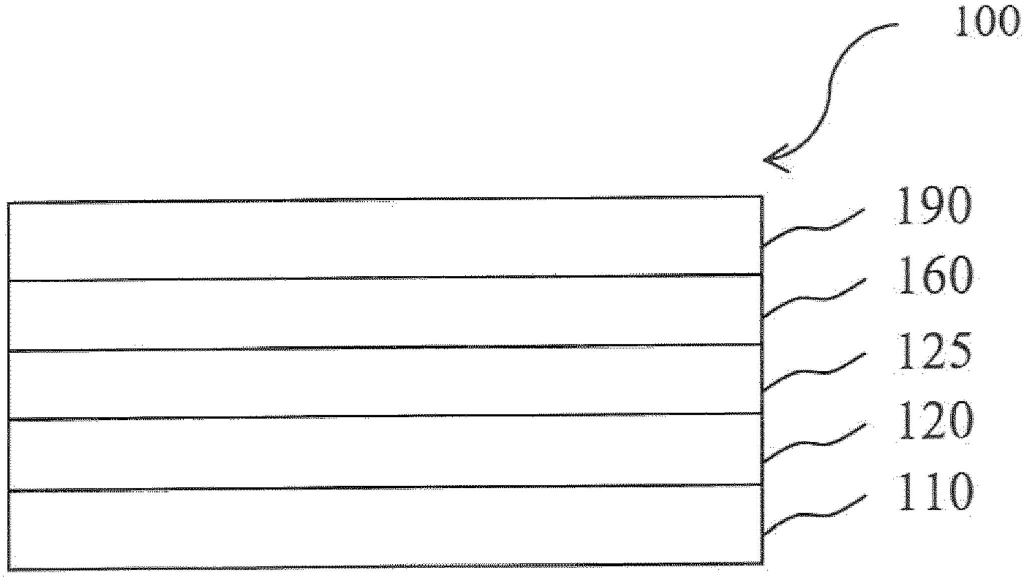


Fig.1

EP 4 321 511 A1

## Description

TECHNICAL FIELD

The present invention relates to a compound, an organic semiconducting material comprising the same, an organic electronic device comprising the same and a method for preparing the organic electronic device.

BACKROUND OF THE INVENTION

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of an organic material of the organic semiconductor layer.

Particularly, development of an organic semiconductor layer being capable of increasing electron mobility and simultaneously increasing electrochemical stability is needed so that the organic electronic device, such as an organic light emitting diode, may be applied to a large-size flat panel display.

Further, development of an organic semiconductor layer being capable to have an extended life span at higher current density and thereby at higher brightness is needed. In particular, the development of an organic semiconductor material or semiconductor layer is needed with respect to lowering the operating voltage, which is important for reducing power consumption and increasing battery life, for example of a mobile display device.

It is, therefore, the object of the present invention to provide an organic light emitting diode overcoming drawbacks of the prior art, in particular providing compounds for use in organic semiconducting materials and electronic devices comprising the same helpful to improve driving voltage and/or efficiency thereof.

DISCLOSURE

This object is achieved by a compound of formula (1)

$$Hy^2\text{-}L^1\text{-}Hy^1 \qquad (1)$$

wherein

- $Hy^1$ has the formula (2)

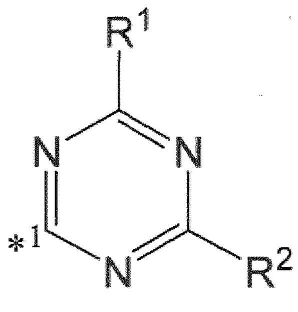

(2)

- $R^1$ and $R^2$ are independently selected from $C_6$ to $C_{60}$ aryl or $C_2$ to $C_{42}$ heteroaryl;

    - $R^1$ and $R^2$ may independently be substituted with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched

alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- each $C_6$ to $C_{12}$ aryl substituent on $R^1$ and/or $R^2$ and each $C_3$ to $C_{11}$ heteroaryl substituent on $R^1$ and/or $R^2$ may independently be substituted with $C_1$ to $C_4$ alkyl or halogen;

- $Hy^2$ has the formula (3)

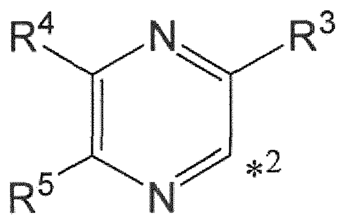
(3)

- wherein $R^3$ to $R^5$ are independently selected from $C_6$ to $C_{60}$ aryl or $C_2$ to $C_{42}$ heteroaryl;

- $R^3$ to $R^5$ may independently be substituted with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_2$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- each $C_6$ to $C_{12}$ aryl substituent on $R_3$ to $R^5$ and each $C_3$ to $C_{11}$ heteroaryl substituent on $R^3$ to $R^5$ may independently be substituted with $C_1$ to $C_4$ alkyl or halogen;

- at least one of $R^3$ to $R^5$ has the formula (4)

$$*^3\text{-}L^2\text{-}Hy^3 \qquad (4)$$

- $*^3\text{-}L^2\text{-}Hy^3$ is bond at "*3" to the remaining structure of formula (3);

- $L^2$ is a single bond or phenylene;

- $Hy^3$ is $C_3$ to $C_{42}$ heteroaryl comprising at least one unsubstituted 6-membered N-containing heteroaromatic ring, wherein $L^2$ and $Hy^3$ are bonded via a single bond:

  - between $L^2$ and the 6-membered N-containing heteroaromatic ring; or

  - between $L^2$ and a condensed ring system with two or three condensed rings, wherein one of the two or three rings is the 6-membered N-containing heteroaromatic ring;

- $L^1$ is selected from substituted or unsubstituted $C_6$ to $C_{60}$ arylene;

  - $L^1$ may be substituted with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from 0, S or Se, preferably O and $R^{10}$ is independently

selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- each $C_6$ to $C_{12}$ aryl substituent on L and each $C_3$ to $C_{11}$ heteroaryl substituent on L may independently be substituted with $C_1$ to $C_4$ alkyl or halogen;

- $L^1$ comprises at least one 6-membered aromatic ring, wherein the 6-membered aromatic ring has the formula (5) or (6)

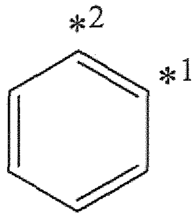
(5)

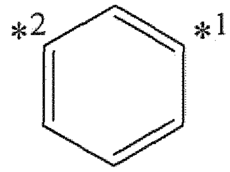
(6);

$Hy^1$ is bonded at "$*^1$" via a single bond to $L^1$; and

$Hy^2$ is bonded at "$*^2$" via a single bond to $L^2$;

wherein the following compounds are excluded:

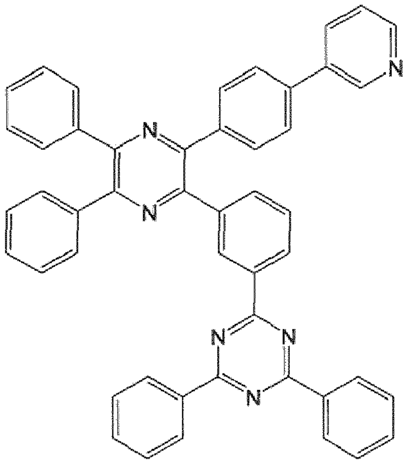
;

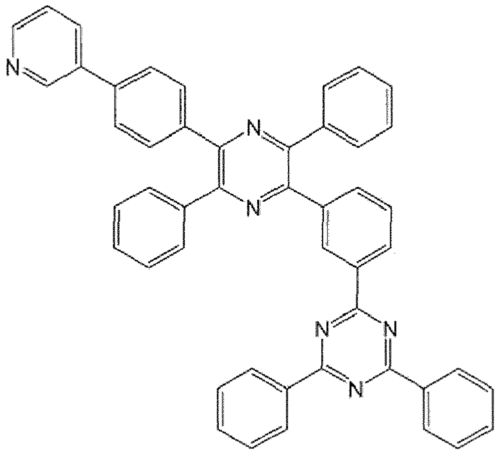
;

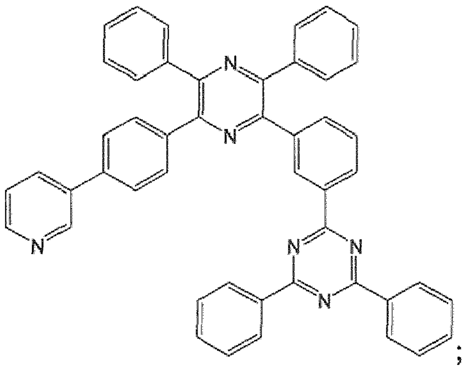
; and

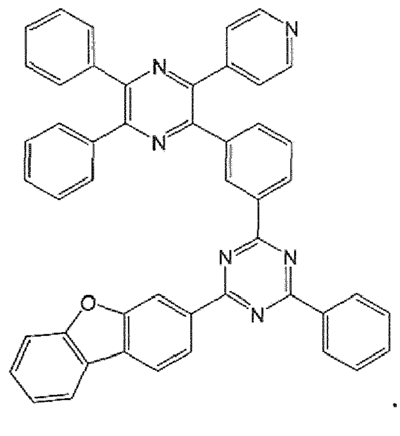
.

The object is further achieved by an organic electronic semiconducting material comprising the compound in accordance with the invention.

The object is further achieved by an organic electronic device comprising a semiconducting layer, wherein the semiconducting layer comprises the compound according to the invention.

The object is further achieved by a display device comprising an organic electronic device comprising a semiconducting layer, wherein the semiconducting layer comprises the compound according to the invention.

Surprisingly, it was found that the organic electronic device according to the invention solves the problem underlying the present invention by enabling devices in various aspects superior over the organic electroluminescent devices known in the art, in particular with respect to efficiency and/or operating voltage.

<u>Compound</u>

According to one aspect the invention is related to a compound of the following formula (1)

$$Hy^2\text{-}L^1\text{-}Hy^1 \qquad (1).$$

In the compound of formula (1), $Hy^1$ has the following formula (2)

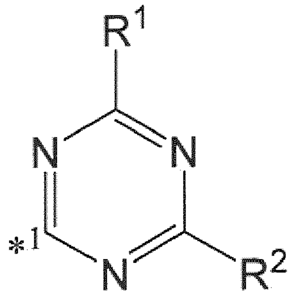

(2).

$Hy^1$ is bonded at "$*^1$" via a single bond to $L^1$.

In formula (2), $R^1$ and $R^2$ are independently selected from $C_6$ to $C_{60}$ aryl or $C_2$ to $C_{42}$ heteroaryl. $R^1$ and $R^2$ may be selected independently from $C_6$ to $C_{54}$ aryl or $C_2$ to $C_{39}$ heteroaryl. $R^1$ and $R^2$ may be selected independently from $C_6$ to $C_{48}$ aryl or $C_2$ to $C_{36}$ heteroaryl. $R^1$ and $R^2$ may be selected independently from $C_6$ to $C_{42}$ aryl or $C_2$ to $C_{33}$ heteroaryl. $R^1$ and $R^2$ may be selected independently from $C_6$ to $C_{36}$ aryl or $C_2$ to $C_{30}$ heteroaryl. $R^1$ and $R^2$ may be selected independently from $C_6$ to $C_{30}$ aryl or $C_2$ to $C_{24}$ heteroaryl. $R^1$ and $R^2$ may be selected independently from $C_6$ to $C_{24}$ aryl or $C_2$ to $C_{21}$ heteroaryl. $R^1$ and $R^2$ may be selected independently from $C_6$ to $C_{18}$ aryl or $C_2$ to $C_{17}$ heteroaryl. $R^1$ and $R^2$ may be selected independently from $C_6$ to $C_{12}$ aryl or $C_2$ to $C_{11}$ heteroaryl.

$R^1$ and $R^2$ may be independently selected from unsubstituted $C_6$ to $C_{60}$ aryl. $R^1$ and $R^2$ may be selected independently from $C_6$ to $C_{54}$ aryl. $R^1$ and $R^2$ may be selected independently from $C_6$ to $C_{48}$ aryl. $R^1$ and $R^2$ may be selected independently from $C_6$ to $C_{42}$ aryl. $R^1$ and $R^2$ may be selected independently from $C_6$ to $C_{36}$ aryl. $R^1$ and $R^2$ may be selected independently from $C_6$ to $C_{30}$ aryl. $R^1$ and $R^2$ may be selected independently from $C_6$ to $C_{24}$ aryl. $R^1$ and $R^2$ may be selected independently from $C_6$ to $C_{18}$ aryl.

$R^1$ and $R^2$ may be selected independently from the group consisting of: phenyl, naphtyl; phenanthrenyl, anthracenyl; fluorenyl; biphenyl; terphenyl; naphthylphenyl; phenylnaphthyl, pyridinyl, quinolinyl, phenanthridinyl, phenylquinolinyl, phenylphenanthridinyl, imidazolyl, benzimidazolyl, furanyl, benzofuranyl, and dibenzofuranyl.

$R^1$ and $R^2$ may be selected independently from the group consisting of: phenyl, naphtyl; phenanthrenyl, anthracenyl; fluorenyl; biphenyl; terphenyl; naphthylphenyl; and phenylnaphthyl.

$R^1$ and $R^2$ may be selected independently from the group consisting of phenyl and biphenyl.

$R^1$ and $R^2$ may be both phenyl.

$R^1$ and $R^2$ in any of the above embodiments may independently be substituted with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy; and each $C_6$ to $C_{12}$ aryl substituent on $R^1$ and/or $R^2$ and each $C_3$ to $C_{11}$ heteroaryl substituent on $R^1$ and/or $R^2$ may independently be substituted with $C_1$ to $C_4$ alkyl or halogen

In the formula (1), $Hy^2$ has the formula (3)

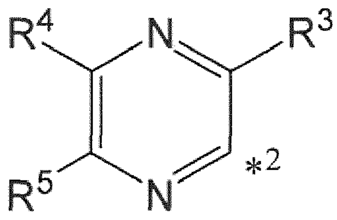

(3).

$Hy^2$ is bonded at "*2" via a single bond to $L^2$.

In the formula 3, $R^3$ to $R^5$ are independently selected from $C_6$ to $C_6$, aryl or $C_2$ to $C_{42}$ heteroaryl. $R^3$ to $R^5$ may be selected independently from $C_6$ to $C_{54}$ aryl or $C_2$ to $C_{39}$ heteroaryl. $R^3$ to $R^5$ may be selected independently from $C_6$ to $C_{48}$ aryl or $C_2$ to $C_{36}$ heteroaryl. $R^3$ to $R^5$ may be selected independently from $C_6$ to $C_{42}$ aryl or $C_2$ to $C_{33}$ heteroaryl. $R^3$ to $R^5$ may be selected independently from $C_6$ to $C_{36}$ aryl or $C_2$ to $C_{30}$ heteroaryl. $R^3$ to $R^5$ may be selected independently from $C_6$ to $C_{30}$ aryl or $C_2$ to $C_{24}$ heteroaryl. $R^3$ to $R^5$ may be selected independently from $C_6$ to $C_{24}$ aryl or $C_2$ to $C_{21}$ heteroaryl. $R^3$ to $R^5$ may be selected independently from $C_6$ to $C_{18}$ aryl or $C_2$ to $C_{17}$ heteroaryl. $R^3$ to $R^5$ may be selected independently from $C_6$ to $C_{12}$ aryl or $C_2$ to $C_{11}$ heteroaryl.

$R^3$ to $R^5$ may in each of the above embodiments independently be substituted with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy; and each $C_6$ to $C_{12}$ aryl substituent on $R^3$ to $R^5$ and each $C_3$ to $C_{11}$ heteroaryl substituent on $R^3$ to $R^5$ may independently be substituted with $C_1$ to $C_4$ alkyl or halogen.

It is provided that at least one of the $R^3$ to $R^5$ has the formula (4)

$$*^3\text{-}L^2\text{-}Hy^3 \qquad (4).$$

It may be provided that $R^3$ and $R^5$ are independently selected from $C_6$ to $C_{60}$ aryl and $R^4$ has the formula (4).

$*^3$-$L^2$-$Hy^3$ is bond at "*3" to the remaining structure of formula (3). That is, "*3" as used in the formula is no group but only designates the position of binding. In this regard, the "remaining structure" refers especially to the pyrazine ring of formula (3)

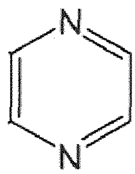

pyrazine

to which the $R^3$ to $R^5$ having the formula (4) is bonded.

$L^2$ is a single bond or phenylene. $L^2$ may be a single bond, meta-phenylene or ortho-phenylene. $L^2$ may be a single bond or meta-phenylene. $L^2$ may be a single bond.

In case that $L^2$ is a single bond, the group of formula (4) is $*^3$-$Hy_3$. For example, in case that $L^2$ is a single bond and $R^4$ is the group of formula 4, formula (3) has the following structure

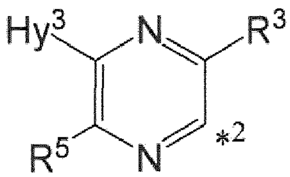

.

In case that $L^2$ is phenylene, the group of formula (4) is $*^3$-phenylene-$Hy^3$. For example, in case that $L^2$ is a meta-phenylene and $R^4$ is the group of formula 4, formula (3) has the following structure

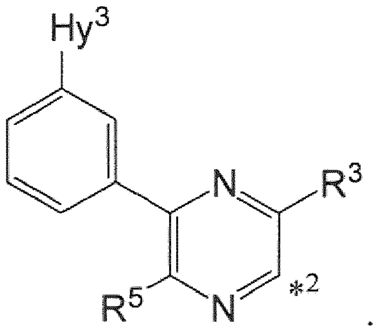

$Hy^3$ is $C_3$ to $C_{42}$ heteroaryl comprising at least one unsubstituted 6-membered N-containing heteroaromatic ring. $Hy^3$ may be $C_3$ to $C_{36}$ heteroaryl comprising at least one unsubstituted 6-membered N-containing heteroaromatic ring. $Hy^3$ may be $C_3$ to $C_{30}$ heteroaryl comprising at least one unsubstituted 6-membered N-containing heteroaromatic ring. $Hy^3$ may be $C_3$ to $C_{24}$ heteroaryl comprising at least one unsubstituted 6-membered N-containing heteroaromatic ring. $Hy^3$ may be $C_3$ to $C_{17}$ heteroaryl comprising at least one unsubstituted 6-membered N-containing heteroaromatic ring. $Hy^3$ may be $C_3$ to $C_{11}$ heteroaryl comprising at least one unsubstituted 6-membered N-containing heteroaromatic ring. $Hy^3$ may be $C_4$ to $C_{11}$ heteroaryl comprising at least one unsubstituted 6-membered N-containing heteroaromatic ring.

The term "unsubstituted" as used in the definition of $Hy^3$ with respect to the 6-membered N-containing heteroaromatic ring does not exclude that the 6-membered N-containing heteroaromatic ring is part of a condensed ring system. For example, in isoquinoline,

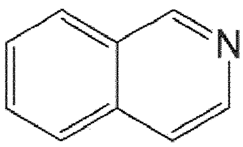
isoquinoline

the N-containing ring (right ring) is unsubstituted in terms of the present disclosure.

The term "unsubstituted" as used in the definition of $Hy^3$ with respect to the 6-membered N-containing heteroaromatic ring does not exclude that the 6-membered N-containing heteroaromatic ring is part of a larger heteroaryl group. For example, if the formula (4) has the following structure,

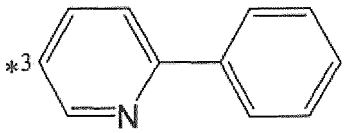

the phenyl ring is not considered as a substituent but as a part of the $C_{11}$ heteroaryl moiety forming together with the N-containing ring the whole $C_{11}$ heteroaryl moiety.

Examples of a "substituted" 6-membered N-containing heteroaromatic ring within the definition of $Hy^3$ which is not in accordance with the invention are, for example, the following structures

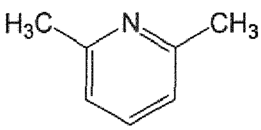 ; 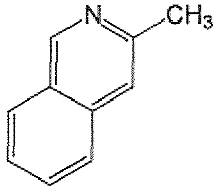 ; 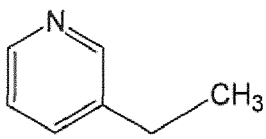

etc.

The term "unsubstituted" as used in the definition of $Hy^3$ with respect to the 6-membered N-containing heteroaromatic ring may mean not substituted with one or more substituents independently selected from the group consisting of $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$

cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy. Especially, "unsubstituted" as used in the definition of $Hy^3$ with respect to the 6-membered N-containing heteroaromatic ring may have the meaning not substituted with $C_1$ to $C_6$ alkyl, especially may have the meaning not substituted with methyl.

$L^2$ and $Hy^3$ are bonded via a single bond either

(1.) between $L^2$ and the 6-membered N-containing heteroaromatic ring;
or

(2.) between $L^2$ and a condensed ring system with two or three condensed rings, wherein one of the two or three rings is the 6-membered N-containing heteroaromatic ring. $L^2$ and $Hy^3$ being bonded via a single bond means in this regard that in case that $L_2$ itself is a single bond than $Hy^3$ is directly bonded to the remaining structure of formula (4), that is, the pyrazine part thereof as shown above.

As an example of (1.), if $Hy^3$ has the following structure

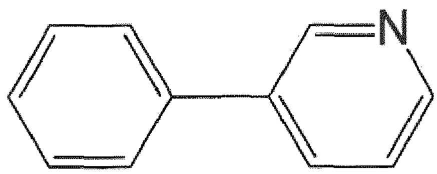

then the bonding to $L^2$ may be via a single bond at each binding position of the N-containing ring (right ring) but not via a binding position of the non-N-containing ring (left ring), wherein it is provided in this example that the left ring does not represent $L^2$.

As an example of (2.), if $Hy^3$ is isoquinoline,

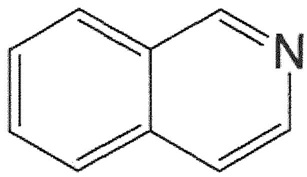
isoquinoline

then the bonding to $L^2$ may be via a single bond at each binding position of the N-containing ring (right ring) and likewise at each binding position of the non-N-containing ring (left ring).

$Hy^3$ may be (especially if $L^2$ is ortho-phenylene or meta-phenylene) $C_3$ to $C_{42}$ heteroaryl comprising at least one structural fragment represented by one of the following formulas (7a) to (7d).

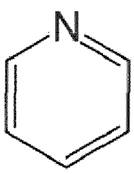 (7a) 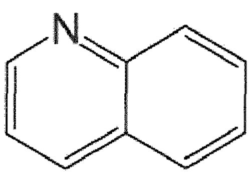 (7b) 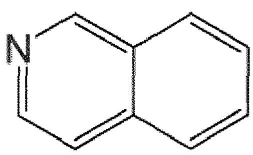 (7c) 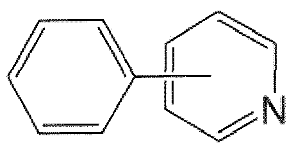 (7d)

$Hy^3$ may be (especially if $L^2$ is ortho-phenylene or meta-phenylene) $C_3$ to $C_{36}$ heteroaryl comprising at least one structural fragment represented by one of the formulas (7a) to (7d). Hy3 may be (especially if $L^2$ is ortho-phenylene or meta-phenylene) $C_3$ to $C_{30}$ heteroaryl comprising at least one structural fragment represented by one of the formulas (7a) to (7d). Hy3 may be (especially if $L^2$ is ortho-phenylene or meta-phenylene) $C_3$ to $C_{24}$ heteroaryl comprising at least one structural fragment represented by one of the formulas (7a) to (7d). $Hy^3$ may be (especially if $L^2$ is ortho-phenylene or meta-phenylene) $C_3$ to $C_{17}$ heteroaryl comprising at least one structural fragment represented by one of the formulas (7a) to (7d). Hy3 may be (especially if $L^2$ is ortho-phenylene or meta-phenylene) $C_3$ to $C_{11}$ heteroaryl comprising at least one structural fragment represented by one of the formulas (7a) to (7d). $Hy^3$ may be (especially if $L^2$ is ortho-phenylene

or meta-phenylene) $C_4$ to $C_{11}$ heteroaryl comprising at least one structural fragment represented by one of the formulas (7a) to (7d).

Hy3 may be (especially if $L^2$ is a single bond) $C_3$ to $C_{42}$ heteroaryl comprising at least one structural fragment represented by one of the following formulas (8a) to (8e)

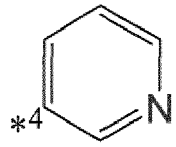
(8a);
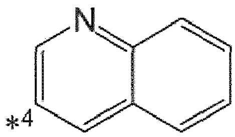
(8b);
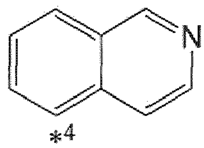
(8c);
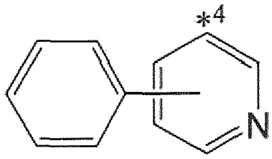
(8d)
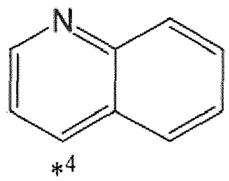
(8e)

wherein $*^4$ represents the position of the single bond by which Hy3 is bonded to $L^2$.

$Hy^3$ may be (especially if $L^2$ is a single bond) $C_3$ to $C_{30}$ heteroaryl comprising at least one structural fragment represented by one of the formulas (8a) to (8e). $Hy^3$ may be (especially if $L^2$ is a single bond) $C_3$ to $C_{24}$ heteroaryl comprising at least one structural fragment represented by one of the formulas (8a) to (8e). $Hy^3$ may be (especially if $L^2$ is a single bond) $C_3$ to $C_{17}$ heteroaryl comprising at least one structural fragment represented by one of the formulas (8a) to (8e). $Hy^3$ may be (especially if $L^2$ is a single bond) $C_3$ to $C_{11}$ heteroaryl comprising at least one structural fragment represented by one of the formulas (8a) to (8e). $Hy^3$ maybe (especially if $L^2$ is a single bond) $C_4$ to $C_{11}$ heteroaryl comprising at least one structural fragment represented by one of the formulas (8a) to (8e).

The structure according to formula (4) may be selected from the following formulas (7) to (11)

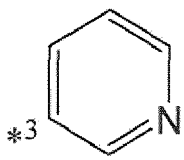
(7);
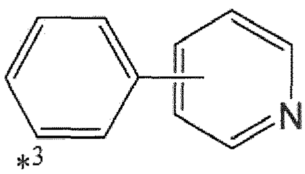
(8);
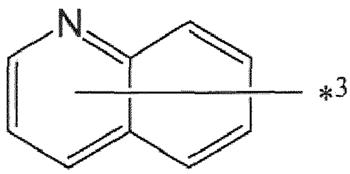
(9);
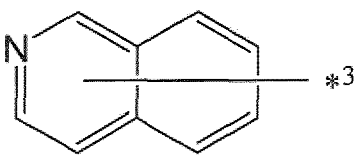
(10);
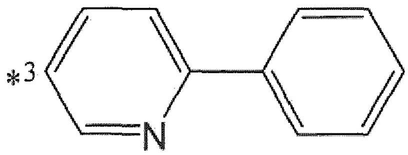
(11).

$R^3$ and $R^5$ maybe independently selected from $C_6$ to $C_{60}$ aryl and at the same time $R^4$ may have the formula (4) and the structure according to formula (4) is selected from the following formulas (7) to (11)

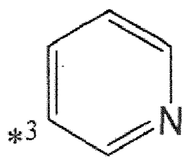
(7);
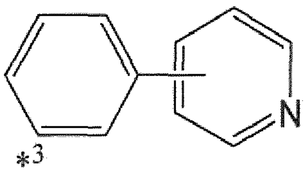
(8);
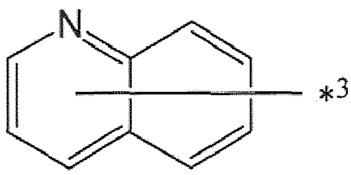
(9);

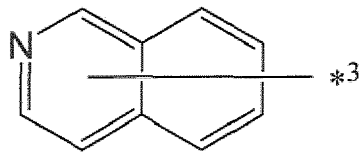

(10);

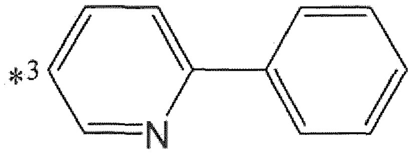

(11).

$L^2$ may be a single bond and at the same time Hy3 is $C_3$ to $C_{42}$ heteroaryl comprising at least one structural fragment represented by one of the following formulas (8a) to (8e)

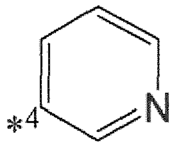

(8a);

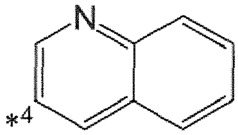

(8b);

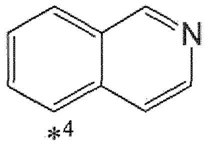

(8c);

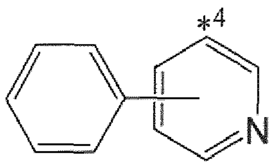

(8d)

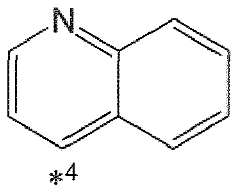

(8e)

wherein L2 and $Hy^3$ are bonded via a single bond and $*^4$ represents the position by which $Hy^3$ is bonded to $L^2$ via the single bond;

or

- $L^2$ may be ortho-phenylene or meta-phenylene, especially meta-phenylene, and at the same time $Hy^3$ is $C_3$ to $C_{42}$ heteroaryl comprising at least one unsubstituted 6-membered N-containing heteroaromatic ring, wherein $L^2$ and $Hy^3$ are bonded via a single bond

- between $L^2$ and the 6-membered N-containing heteroaromatic ring; or

- between $L^2$ and a condensed ring system with two or three condensed rings, wherein one of the two or three rings is the 6-membered N-containing heteroaromatic ring.

$L^2$ may be a single bond and at the same time Hy3 is $C_3$ to $C_{30}$ heteroaryl comprising at least one structural fragment represented by one of the following formulas (8a) to (8e)

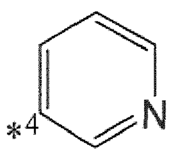

(8a);

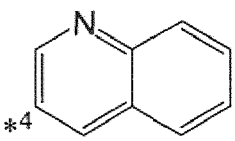

(8b);

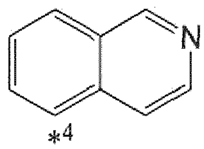

(8c);

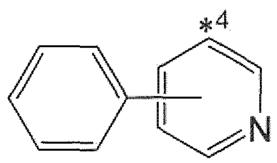

(8d)

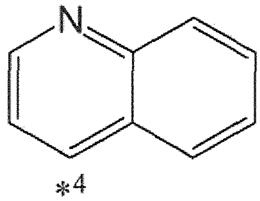

(8e)

wherein $L^2$ and $Hy^3$ are bonded via a single bond and $*^4$ represents the position by which $Hy^3$ is bonded to $L^2$ via

the single bond;
or

- $L^2$ may be ortho-phenylene or meta-phenylene, especially meta-phenylene, and at the same time $Hy^3$ is $C_3$ to $C_{42}$ heteroaryl comprising at least one unsubstituted 6-membered N-containing heteroaromatic ring, wherein $L^2$ and $Hy^3$ are bonded via a single bond

- between $L^2$ and the 6-membered N-containing heteroaromatic ring; or

- between $L^2$ and a condensed ring system with two or three condensed rings, wherein one of the two or three rings is the 6-membered N-containing heteroaromatic ring.

$L^2$ may be a single bond and at the same time $Hy^3$ is $C_3$ to $C_{24}$ heteroaryl comprising at least one structural fragment represented by one of the following formulas (8a) to (8E)

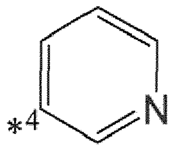 (8a); 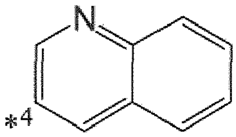 (8b); 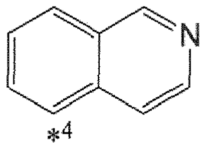 (8c);

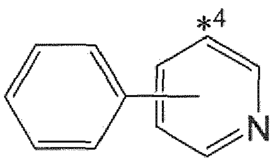 (8d) 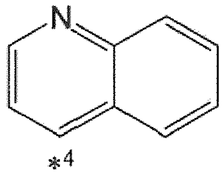 (8e)

wherein $L^2$ and $Hy^3$ are bonded via a single bond and *4 represents the position by which $Hy^3$ is bonded to $L^2$ via the single bond;
or

- $L^2$ may be ortho-phenylene or meta-phenylene, especially meta-phenylene, and at the same time $Hy^3$ is $C_3$ to $C_{42}$ heteroaryl comprising at least one unsubstituted 6-membered N-containing heteroaromatic ring, wherein $L^2$ and Hy3 are bonded via a single bond

- between $L^2$ and the 6-membered N-containing heteroaromatic ring; or

- between $L^2$ and a condensed ring system with two or three condensed rings, wherein one of the two or three rings is the 6-membered N-containing heteroaromatic ring.

$L^2$ may be a single bond and at the same time $Hy^3$ is $C_3$ to $C_{17}$ heteroaryl comprising at least one structural fragment represented by one of the following formulas (8a) to (8e)

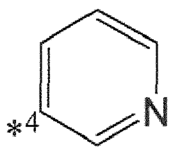 (8a); 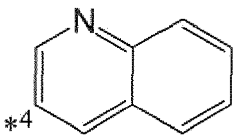 (8b); 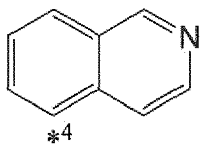 (8c);

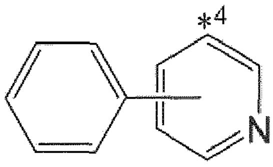 (8d) 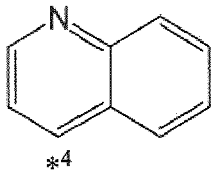 (8e)

wherein $L^2$ and $Hy^3$ are bonded via a single bond and *4 represents the position by which $Hy^3$ is bonded to $L^2$ via the single bond;
or

- $L^2$ may be ortho-phenylene or meta-phenylene, especially meta-phenylene, and at the same time Hy3 is $C_3$ to $C_{42}$ heteroaryl comprising at least one unsubstituted 6-membered N-containing heteroaromatic ring, wherein $L^2$ and $Hy^3$ are bonded via a single bond

- between $L^2$ and the 6-membered N-containing heteroaromatic ring; or

- between $L^2$ and a condensed ring system with two or three condensed rings, wherein one of the two or three rings is the 6-membered N-containing heteroaromatic ring.

The structure according to formula (4) may be selected from the following formulas (7), (11) and (9a) to (9q)

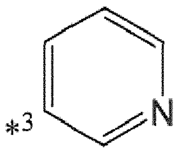 (7); 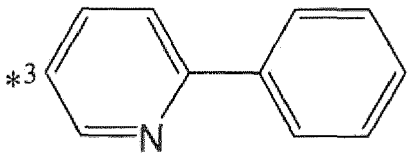 (11); 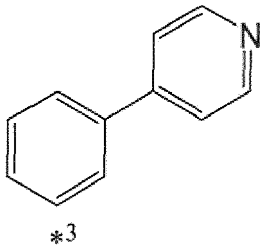 (9a);

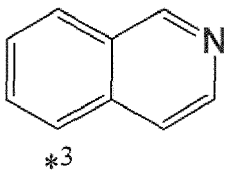 (9b); 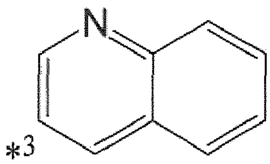 (9c); 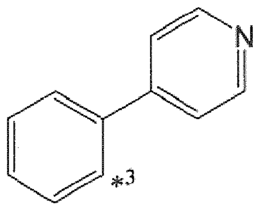 (9d);

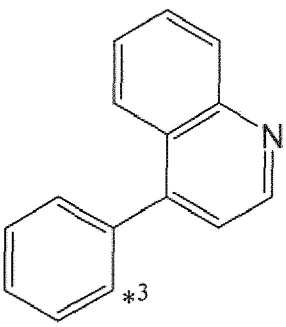 (9e); 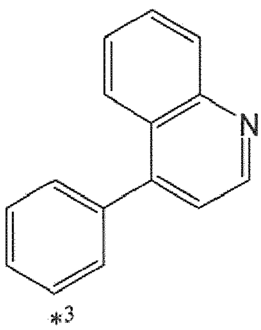 (9f); 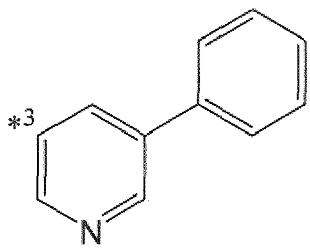 (9g) ;

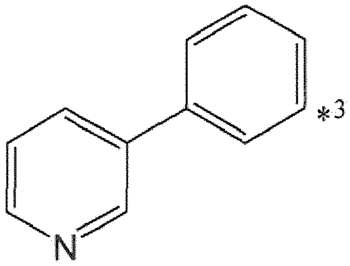 (9h); 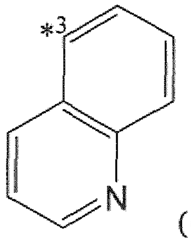 (9i); 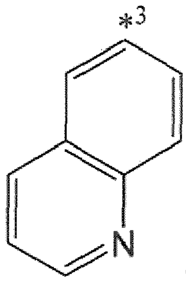 (9j); 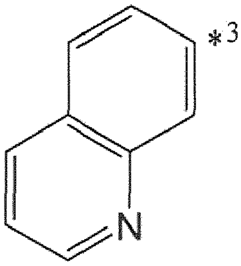 (9k);

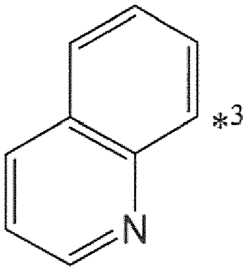 (9l); 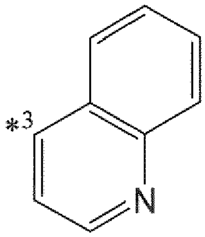 (9m); 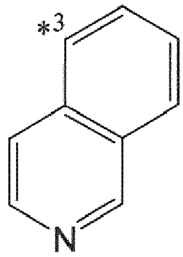 (9n); 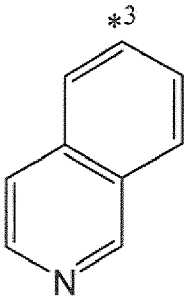 (9o);

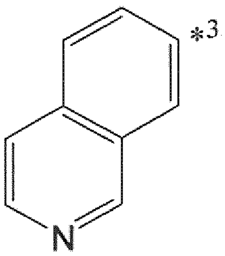 (9p); 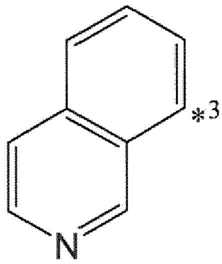 (9q).

R3 and $R^5$ maybe independently selected from $C_6$ to $C_{60}$ aryl and at the same time $R^4$ may have the formula (4) and the structure according to formula (4) may be selected from the following formulas (7), (11) and (9a) to (9q)

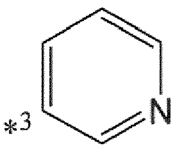 (7); 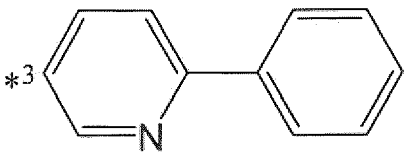 (11); 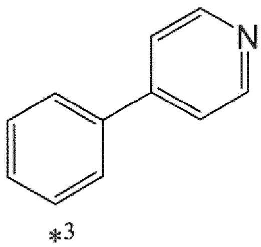 (9a);

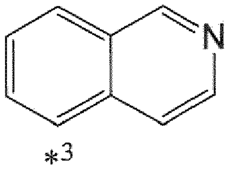 (9b); 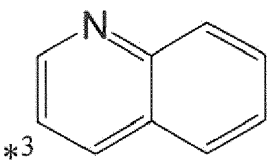 (9c) ; 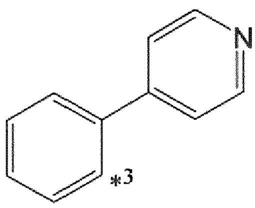 (9d);

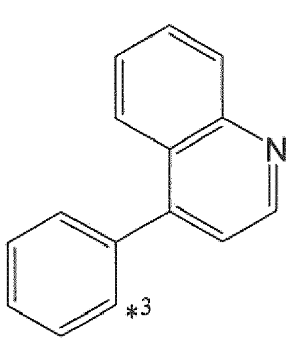 (9e); 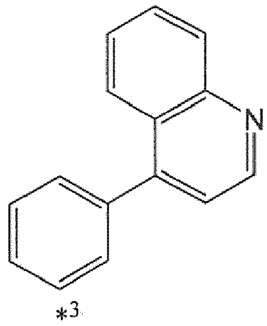 (9f); 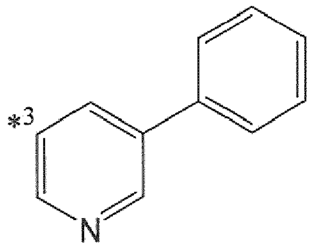 (9g);

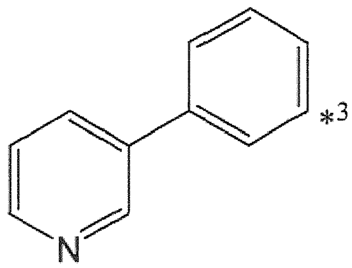 (9h); 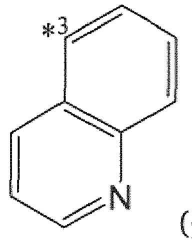 (9i); 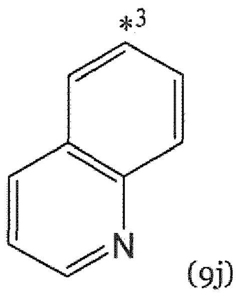 (9j); 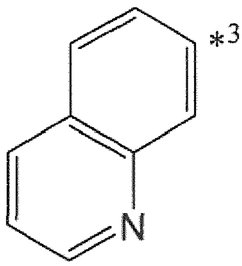 (9k);

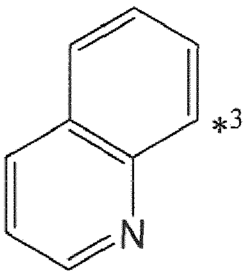 (9l); 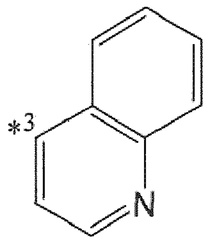 (9m); 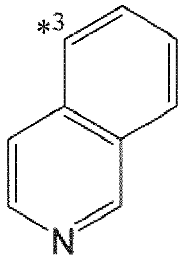 (9n); 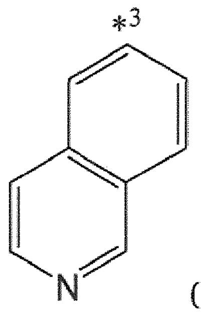 (9o);

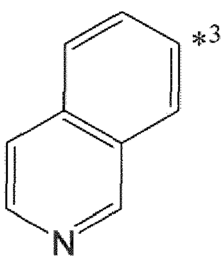 (9p); 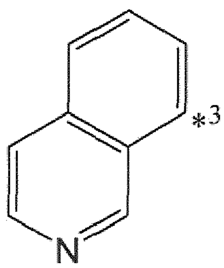 (9q).

$L^1$ is selected from substituted or unsubstituted $C_6$ to $C_{60}$ arylene. $L^1$ comprises at least one 6-membered aromatic ring, wherein the 6-membered aromatic ring has the formula (5) or (6)

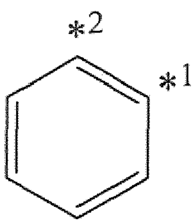 (5) 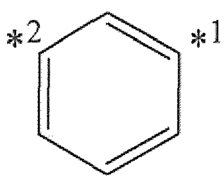 (6)

$L^1$ may be selected from substituted or unsubstituted $C_6$ to $C_{54}$ arylene and the 6-membered aromatic ring has the formula (5) or (6). $L^1$ may be selected from substituted or unsubstituted $C_6$ to $C_{48}$ arylene and the 6-membered aromatic ring has the formula (5) or (6). $L^1$ may be selected from substituted or unsubstituted $C_6$ to $C_{42}$ arylene and the 6-membered aromatic ring has the formula (5) or (6). $L^1$ may be selected from substituted or unsubstituted $C_6$ to $C_{36}$ arylene and the 6-membered aromatic ring has the formula (5) or (6). $L^1$ may be selected from substituted or unsubstituted $C_6$ to $C_{30}$ arylene and the 6-membered aromatic ring has the formula (5) or (6). $L^1$ may be selected from substituted or unsubstituted $C_6$ to $C_{24}$ arylene and the 6-membered aromatic ring has the formula (5) or (6). $L^1$ may be selected from substituted or unsubstituted $C_6$ to $C_{18}$ arylene and the 6-membered aromatic ring has the formula (5) or (6). $L^1$ may be selected from substituted or unsubstituted $C_6$ to $C_{12}$ arylene and the 6-membered aromatic ring has the formula (5) or (6). $L^1$ may be selected from substituted or unsubstituted $C_6$ to $C_{10}$ arylene and the 6-membered aromatic ring has the formula (5) or (6).

$L^1$ may be selected from substituted or unsubstituted $C_6$ to $C_{54}$ arylene and the 6-membered aromatic ring has the formula (6). $L^1$ may be selected from substituted or unsubstituted $C_6$ to $C_{48}$ arylene and the 6-membered aromatic ring has the formula (6). $L^1$ may be selected from substituted or unsubstituted $C_6$ to $C_{42}$ arylene and the 6-membered aromatic ring has the formula (6). $L^1$ may be selected from substituted or unsubstituted $C_6$ to $C_{36}$ arylene and the 6-membered aromatic ring has the formula (6). $L^1$ may be selected from substituted or unsubstituted $C_6$ to $C_{30}$ arylene and the 6-membered aromatic ring has the formula (6). $L^1$ may be selected from substituted or unsubstituted $C_6$ to $C_{24}$ arylene and the 6-membered aromatic ring has the formula (6). $L^1$ may be selected from substituted or unsubstituted $C_6$ to $C_{18}$ arylene and the 6-membered aromatic ring has the formula (6). $L^1$ may be selected from substituted or unsubstituted $C_6$ to $C_{12}$ arylene and the 6-membered aromatic ring has the formula (6). $L^1$ may be selected from substituted or unsubstituted $C_6$ to $C_{10}$ arylene and the 6-membered aromatic ring has the formula (6).

$L^1$ may be substituted in each of the above embodiments with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy; and each $C_6$ to $C_{12}$ aryl substituent on L and each $C_3$ to $C_{11}$ heteroaryl substituent on L may independently be substituted with $C_1$ to $C_4$ alkyl or halogen.

The 6-membered aromatic ring comprised in $L^1$ may have the formula (6)

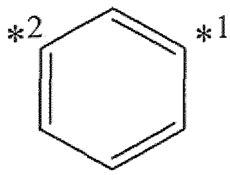

(6).

$L^1$ may have a formula selected from the following formulas (13) to (16)

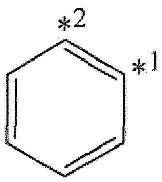

(13);

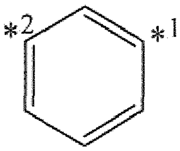

(14);

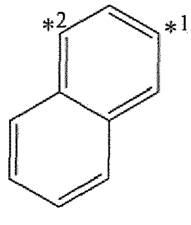

(15); and

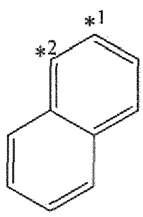

(16).

The compound of formula (1) may have a |LUMO - LUMO+1| ≤ 0.08 eV.

The compound may meet the following requirement: 0.04 ≤ |LUMO - LUMO+1 | ≤ 0.08 eV.

In terms of the present disclosure, the expression "LUMO+1" refers to the first molecular orbital in energy after the LUMO.

According to one embodiment, there a compound of formula (1)

$$Hy^2\text{-}L^1\text{-}Hy^1 \qquad (1)$$

wherein

- $Hy^1$ has the formula (2)

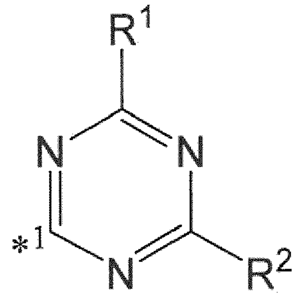

(2)

- $R^1$ and $R^2$ are independently selected from $C_6$ to $C_{60}$ aryl or $C_2$ to $C_{42}$ heteroaryl;

  - $R^1$ and $R^2$ may independently be substituted with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

  - each $C_6$ to $C_{12}$ aryl substituent on $R^1$ and/or $R^2$ and each $C_3$ to $C_{11}$ heteroaryl substituent on $R^1$ and/or $R^2$ may independently be substituted with $C_1$ to $C_4$ alkyl or halogen;

- $Hy^2$ has the formula (3)

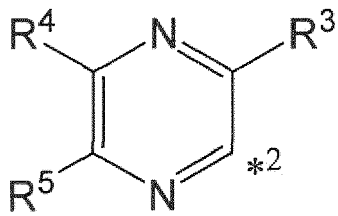

(3)

- wherein $R^3$ to $R^5$ are independently selected from $C_6$ to $C_{60}$ aryl or $C_2$ to $C_{42}$ heteroaryl;

  - $R^3$ to $R^5$ may independently be substituted with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

  - each $C_6$ to $C_{12}$ aryl substituent on $R^3$ to $R^5$ and each $C_3$ to $C_{11}$ heteroaryl substituent on $R^3$ to $R^5$ may independently be substituted with $C_1$ to $C_4$ alkyl or halogen;

- at least one of $R^3$ to $R^5$ has the formula (4)

$$*^3\text{-}L^2\text{-}Hy^3 \qquad (4)$$

- *3-$L^2$-$Hy^3$ is bond at "*3" to the remaining structure of formula (3); wherein

  - $L^2$ is a single bond and $Hy^3$ is $C_3$ to $C_{42}$ heteroaryl comprising at least one structural fragment represented by one of the following formulas (8a) to (8e)

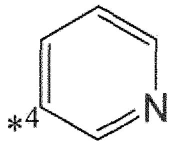 (8a); 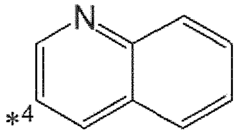 (8b); 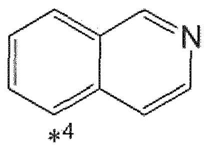 (8c);

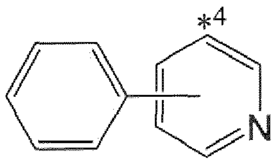 (8d) 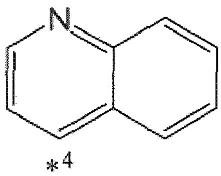 (8e)

  wherein $L^2$ and $Hy^3$ are bonded via a single bond and *4 represents the position of the single bond by which $Hy^3$ is bonded to $L^2$;
  or

  - $L^2$ is ortho-phenylene or meta-phenylene and $Hy^3$ is $C_3$ to $C_{42}$ heteroaryl comprising at least one unsubstituted 6-membered N-containing heteroaromatic ring, wherein $L^2$ and $Hy^3$ are bonded via a single bond

    - between $L^2$ and the 6-membered N-containing heteroaromatic ring; or

    - between $L^2$ and a condensed ring system with two or three condensed rings, wherein one of the two or three rings is the 6-membered N-containing heteroaromatic ring;

- $L^1$ is selected from substituted or unsubstituted $C_6$ to $C_{60}$ arylene;

  - $L^1$ may be substituted with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably 0 and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

  - each $C_6$ to $C_{12}$ aryl substituent on L and each $C_3$ to $C_{11}$ heteroaryl substituent on L may independently be substituted with $C_1$ to $C_4$ alkyl or halogen;

- $L^1$ comprises at least one 6-membered aromatic ring, wherein the 6-membered aromatic ring has the formula (5) or (6)

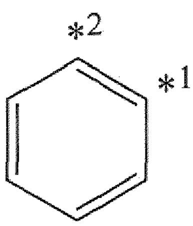 (5) 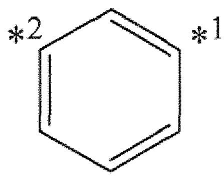 (6);

$Hy^1$ is bonded at "*1" via a single bond to $L^1$; and

$Hy^2$ is bonded at "*2" via a single bond to $L^2$.

According to one embodiment, there a compound of formula (1)

$Hy^2$-$L^1$-$Hy^1$ (1)

wherein

- $Hy^1$ has the formula (2)

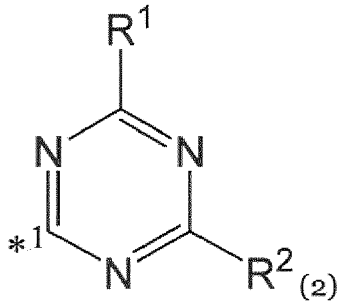
(2)

- $R^1$ and $R^2$ are independently selected from $C_6$ to $C_{60}$ aryl;
  - $R^1$ and $R^2$ may independently be substituted with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - each $C_6$ to $C_{12}$ aryl substituent on $R^1$ and/or $R^2$ and each $C_3$ to $C_{11}$ heteroaryl substituent on $R^1$ and/or $R^2$ may independently be substituted with $C_1$ to $C_4$ alkyl or halogen;
- $Hy^2$ has the formula (3)

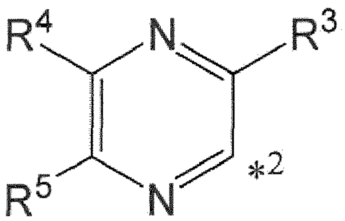
(3)

- wherein R3 and $R^5$ are independently selected from $C_6$ to $C_{60}$ aryl;
  - R3 to $R^5$ may independently be substituted with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - each $C_6$ to $C_{12}$ aryl substituent on $R^3$ to $R^5$ and each $C_3$ to $C_{11}$ heteroaryl substituent on R3 to $R^{5i}$ may independently be substituted with $C_1$ to $C_4$ alkyl or halogen;

- R4 has the formula (4)

$$*^{3}\text{-}L^{2}\text{-}Hy^{3} \qquad (4)$$

- $*^{3}$-$L^2$-$Hy^3$ is bond at "*3" to the remaining structure of formula (3);

  - $L^2$ is a single bond and $Hy^3$ is $C_3$ to $C_{42}$ heteroaryl comprising at least one structural fragment represented by one of the following formulas (8a) to (8e)

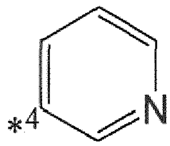
(8a);
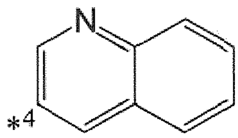
(8b);
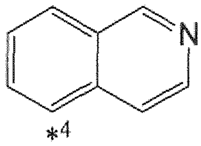
(8c);

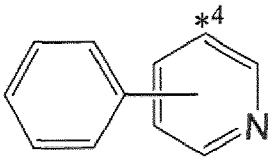
(8d)
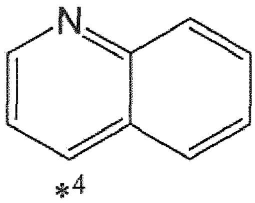
(8e)

  wherein $L^2$ and $Hy^3$ are bonded via a single bond and $*^{4}$ represents the position of the single bond by which $Hy^3$ is bonded to $L^2$;
  or

  - $L^2$ is ortho-phenylene or meta-phenylene and Hy3 is $C_3$ to $C_{42}$ heteroaryl comprising at least one unsubstituted 6-membered N-containing heteroaromatic ring, wherein $L^2$ and $Hy^3$ are bonded via a single bond

    - between $L^2$ and the 6-membered N-containing heteroaromatic ring; or

    - between $L^2$ and a condensed ring system with two or three condensed rings, wherein one of the two or three rings is the 6-membered N-containing heteroaromatic ring;

- $L^1$ is selected from substituted or unsubstituted $C_6$ to $C_{18}$ arylene;

  - $L^1$ may be substituted with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

  - each $C_6$ to $C_{12}$ aryl substituent on L and each $C_3$ to $C_{11}$ heteroaryl substituent on L may independently be substituted with $C_1$ to $C_4$ alkyl or halogen;

- $L^1$ comprises at least one 6-membered aromatic ring, wherein the 6-membered aromatic ring has the formula (5) or (6)

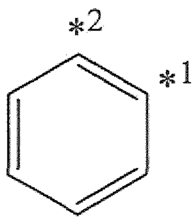 (5) 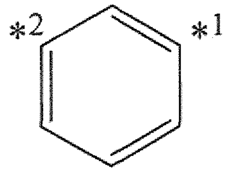 (6);

Hy$^1$ is bonded at "$*^1$" via a single bond to L$^1$; and

Hy$^2$ is bonded at "$*^2$" via a single bond to L$^2$.

According to one embodiment, there a compound of formula (1)

Hy$^2$-L$^1$-Hy$^1$ (1)

wherein

- Hy$^1$ has the formula (2)

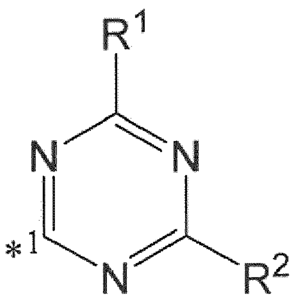 (2)

- R$^1$ and R$^2$ are phenyl;
- Hy$^2$ has the formula (3)

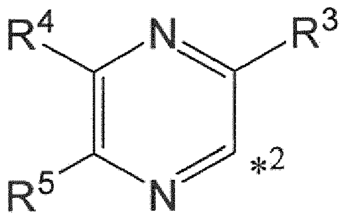 (3)

- wherein R$^3$ and R$^5$ are phenyl;
- R$^4$ is selected from the following formulas (7) to (11)

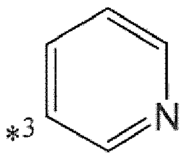 (7); 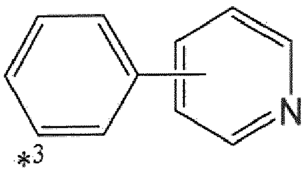 (8); 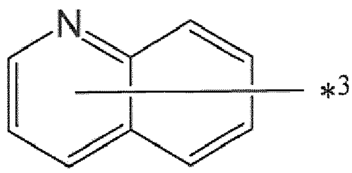 (9);

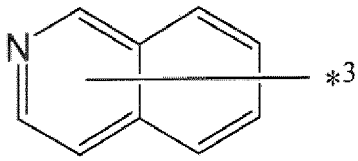

(10);

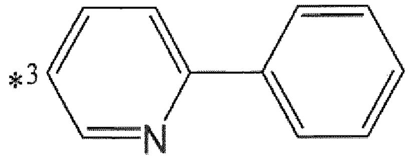

(11).

wherein the respective $R^4$ is bond at "*3" to the remaining structure of formula (3);

- $L^1$ has the formula (5) or (6)

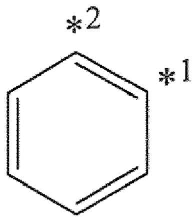

(5)

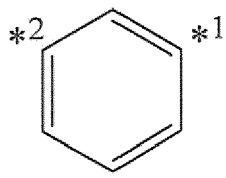

(6);

$Hy^1$ is bonded at "*1" via a single bond to $L^1$; and
$Hy^2$ is bonded at "*2" via a single bond to $L^2$.

The compound of formula (1) may be selected from the following compounds E1 to E6.

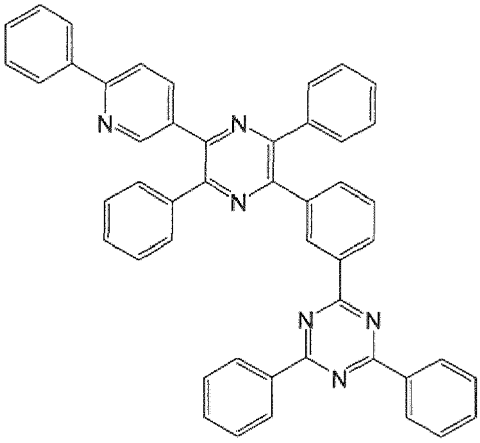

E1

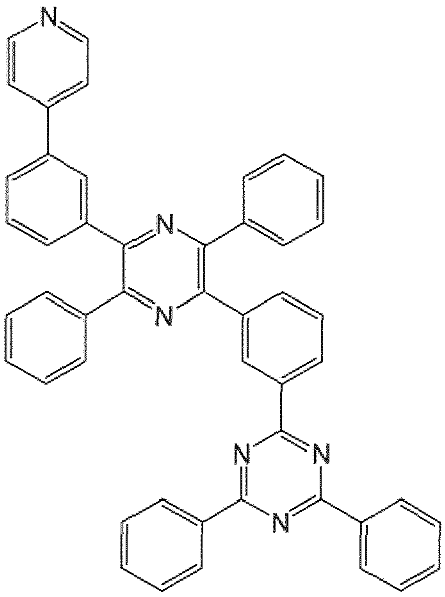

E2

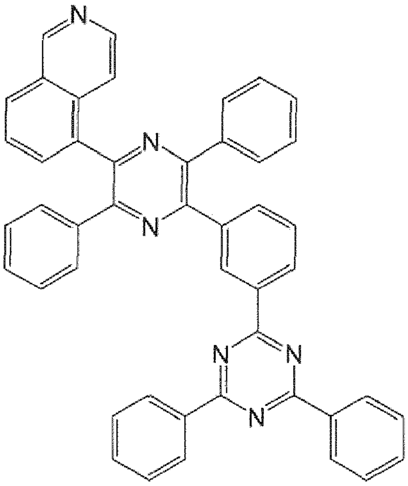
E3

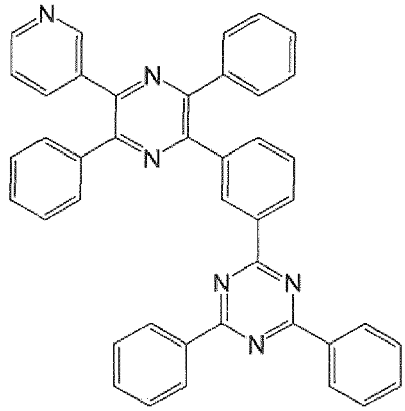
E4

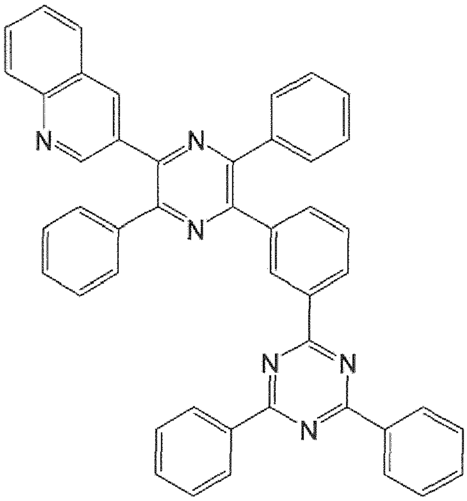
E5

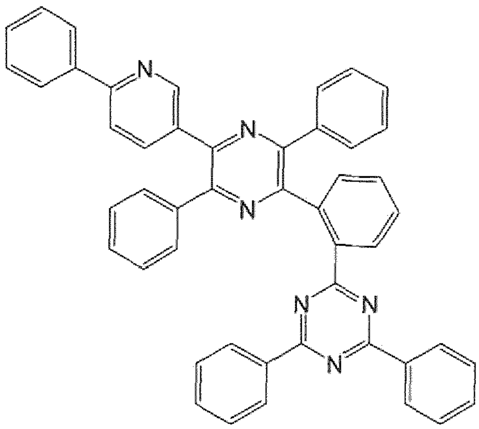
E6.

Organic semiconducting material

The object is further achieved by an organic semiconducting material comprising the compound of formula (1) in accordance with the present invention.

The organic semiconducting material may, in addition to the compound of formula (1) comprise an electrical dopant, such as an n-type dopant, especially a redox n-type dopant.

Undern-type dopant it is understood a compound which, if embedded into an electron transport matrix, improves, in comparison with the neat matrix under the same physical conditions, the electron properties of the formed organic material, particularly in terms of electron injection and/or electron conductivity.

In the context of the present invention "embedded into an electron transport matrix" means homogenously mixed with the electron transport matrix.

The n-type dopant may be selected from elemental metals, metal salts, metal complexes and organic radicals.

In one embodiment, the n-type dopant is selected from alkali metal salts and alkali metal complexes; preferably from lithium salts and lithium organic complexes; more preferably from lithium halides and lithium organic chelates; even more preferably from lithium fluoride, a lithium quinolinolate, lithium borate, lithium phenolate, lithium pyridinolate or from a lithium complex with a Schiff base ligand; most preferably,

- the lithium complex has the formula II, III or IV:

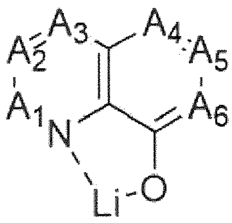 (II), 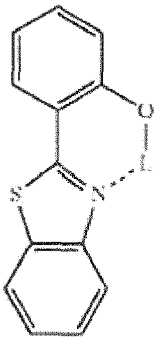 (III), 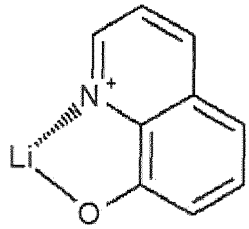 (IV)

wherein

$A_1$ to $A_6$ are same or independently selected from CH, CR, N, O;

R is same or independently selected from hydrogen, halogen, alkyl or aryl or heteroaryl with 1 to 20 carbon atoms; and more preferred $A_1$ to $A_6$ are CH,

- the borate based organic ligand is a tetra(1H-pyrazol-1-yl)borate,
- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
- the pyridinolate is a 2-(diphenylphosphoryl)pyridin-3-olate,
- the lithium Schiff base has the structure 100, 101, 102 or 103:

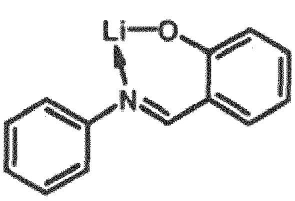 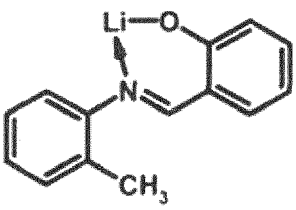 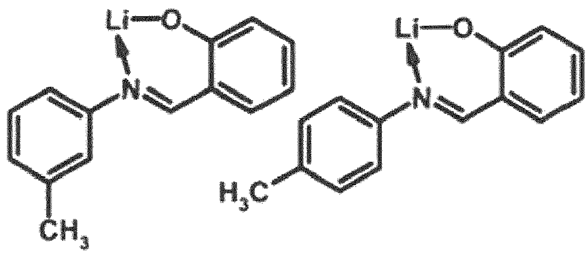

100 101 102 103

According to one embodiment of the invention, the organic semiconducting material of the present invention comprises a lithium organic complex, alternatively 8-Hydroxyquinolinolato-lithium (= LiQ).

According to one embodiment of the present invention the organic semiconducting material comprises a metal, preferably selected from alkali metals, alkaline earth metals, rare earth metals and metals of the first transition period Ti, V, Cr and Mn, especially selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

The most practical benchmark for the strength of an n-dopant is the value of its redox potential. There is no particular limitation in terms how negative the value of the redox potential can be.

As reduction potentials of usual electron transport matrices used in organic semiconductors are, if measured by cyclic voltammetry against ferrocene/ferrocenium reference redox couple, roughly in the range from about - 0.8 V to about - 3.1V; the practically applicable range of redox potentials for n-type dopants which can effectively n-dope such matrices is in a slightly broader range, from about - 0.5 to about - 3.3 V.

The measurement of redox potentials is practically performed for a corresponding redox couple consisting of the reduced and of the oxidized form of the same compound.

In case that the n-type dopant is an electrically neutral metal complex and/or an electrically neutral organic radical, the measurement of its redox potential is actually performed for the redox couple formed by

(i) the electrically neutral metal complex and its cation radical formed by an abstraction of one electron from the

electrically neutral metal complex, or

(ii) the electrically neutral organic radical and its cation formed by an abstraction of one electron from the electrically neutral organic radical.

Preferably, the redox potential of the electrically neutral metal complex and/or of the electrically neutral organic radical may have a value which is more negative than - 0.5 V, preferably more negative than - 1.2 V, more preferably more negative than - 1.7 V, even more preferably more negative than - 2.1 V, most preferably more negative than - 2.5 V, if measured by cyclic voltammetry against ferrocene/ferrocenium reference redox couple for a corresponding redox couple consisting of

(i) the electrically neutral metal complex and its cation radical formed by an abstraction of one electron from the electrically neutral metal complex, or

(ii) the electrically neutral organic radical and its cation formed by an abstraction of one electron from the electrically neutral organic radical.

In a preferred embodiment, the redox potential of the n-dopant is between the value which is about 0.5 V more positive and the value which is about 0.5 V more negative than the value of the reduction potential of the chosen electron transport matrix.

Electrically neutral metal complexes suitable as n-typevdopants may be e.g. strongly reductive complexes of some transition metals in low oxidation state. Particularly strong n-type dopants may be selected for example from Cr(II), Mo(II) and/or W(II) guanidinate complexes such as W2(hpp)4, as described in more detail in WO2005/086251.

Electrically neutral organic radicals suitable as n-type dopants may be e.g. organic radicals created by supply of additional energy from their stable dimers, oligomers or polymers, as described in more detail in EP 1837 926 B1, WO2007/107306, or WO2007/107356. Under an elemental metal, it is understood a metal in a state of a neat metal, of a metal alloy, or in a state of free atoms or metal clusters. It is understood that metals deposited by vacuum thermal evaporation from a metallic phase, e.g. from a neat bulk metal, vaporize in their elemental form. It is further understood that if the vaporized elemental metal is deposited together with a covalent matrix, the metal atoms and/or clusters are embedded in the covalent matrix. In other words, it is understood that any metal doped covalent material prepared by vacuum thermal evaporation contains the metal at least partially in its elemental form.

For the use in consumer electronics, only metals containing stable nuclides or nuclides having very long halftime of radioactive decay might be applicable. As an acceptable level of nuclear stability, the nuclear stability of natural potassium can be taken.

In one embodiment, the n-dopant may be selected from electropositive metals selected from alkali metals, alkaline earth metals, rare earth metals and metals of the first transition period Ti, V, Cr and Mn. Preferably, the n-dopant may be selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.Organic electronic device

The object is further achieved by an organic electronic device comprising a semiconductor layer, wherein the semiconductor layer comprises the compound of formula (1) in accordance with the present invention, especially comprises or consists of the organic semiconducting material according to the invention.

According to one embodiment of the invention, the organic semiconductor layer of the present invention is an electron transport layer, an electron injection layer or charge generation layer; alternatively an electron transport layer or a charge generation layer.

The organic electronic device according to the invention may be an organic light emitting diode.

In accordance with the invention, the organic electronic device may comprise, besides the layers already mentioned above, further layers. Exemplary embodiments of respective layers are described in the following:

*Substrate*

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate maybe both a transparent as well as a non-transparent material, for example a glass substrate, a plastic substrate, a metal substrate or a silicon substrate.

*Anode electrode*

Either a first electrode or a second electrode comprised in the inventive organic electronic device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide ($SnO_2$), aluminum zinc oxide (AlZO) and zinc oxide (ZnO), may be used to form the anode electrode. The anode electrode may also be formed using metals, typically silver (Ag), gold (Au), or metal alloys.

*Hole injection layer*

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10-8 to 10-3 Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluorotetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluorotetracyanoquinonedimethane (F4TCNQ), which LUMO level is about -5.2 eV; zinc phthalocyanine (ZnPc) (HOMO = -5.2 eV) doped with F4TCNQ; $\alpha$-NPD (N,N'-Bis(naphthalen-1-yl)-N,N'-bis(phenyl)-benzidine) doped with F4TCNQ. $\alpha$-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics,

without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.
If the electron blocking layer has a high triplet level, it may also be described as triplet control layer.
The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Photoactive layer (PAL)*

The photoactive layer converts an electrical current into photons or photons into an electrical current.
The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.
It may be provided that the photoactive layer does not comprise the compound of Formula (1).
The photoactive layer may be a light-emitting layer or a light-absorbing layer.

*Emission layer (EML)*

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.
It may be provided that the emission layer does not comprise the compound of Formula (1).
The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).
The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.
Examples of red emitter dopants are PtOEP, Ir(piq)3, and Btp2Ir(acac), but are not limited thereto. These compounds are phosphorescent emitters, however, fluorescent red emitter dopants could also be used.
Examples of phosphorescent green emitter dopants are Ir(ppy)3 (ppy = phenylpyridine), Ir(ppy)2(acac), Ir(mpyp)3.
Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.
The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.Hole *blocking layer (HBL)*
A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function. The hole blocking layer may be the inventive organic semiconductor layer comprising or consisting of the inventive compound represented

by the general Formula (1) as defined above.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, and phenanthroline derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

The hole blocking layer may also be described as a-ETL or auxiliary ETL.

According to an embodiment, a hole blocking layer is arranged between the at least one photoactive layer and the organic semiconductor layer comprising compound of Formula (1).

According to an embodiment, a hole blocking layer is arranged between the at least one photoactive layer and the organic semiconductor layer comprising compound of Formula (1), wherein the organic semiconductor layer comprising compound of Formula (1) further comprises an additive, the additive may be an n-type dopant.

According to an embodiment, a hole blocking layer is arranged between the at least one photoactive layer and the organic semiconductor layer comprising compound of Formula (1), wherein the organic semiconductor layer comprising compound of Formula (1) further comprises a metal, a metal compound or a metal organic complex, alternatively a metal or a lithium organic complex.

According to an embodiment, a hole blocking layer is arranged between the at least one photoactive layer and the organic semiconductor layer comprising compound of Formula (1), wherein the organic semiconductor layer comprising compound of Formula (1) further comprises a metal.

*Electron transport layer (ETL)*

The OLED according to the present invention may comprise an electron transport layer (ETL). In accordance with one preferred embodiment of the invention, the electron transport layer may be the inventive organic semiconductor layer comprising the inventive compound represented by the general Formula (1) as defined herein.

According to various embodiments the OLED may comprise an electron transport layer or an electron transport layer stack comprising at least a first electron transport layer and at least a second electron transport layer.

By suitably adjusting energy levels of particular layers of the ETL, the injection and transport of the electrons may be controlled, and the holes may be efficiently blocked. Thus, the OLED may have long lifetime.

The electron transport layer of the organic electronic device may comprise the compound represented by general Formula (1) as defined above as the organic electron transport matrix (ETM) material. The electron transport layer may comprise, besides or instead of the compound represented by the general Formula (1), further ETM materials known in the art. Likewise, the electron transport layer may comprise as the only electron transport matrix material the compound represented by general Formula (1). In case that the inventive organic electronic device comprises more than one electron transport layers, the compound represented by the general Formula (1) may be comprised in only one of the electron transport layers, in more than one of the electron transport layers or in all of the electron transport layers. In accordance with the invention, the electron transport layer may comprise, besides the ETM material, at least one additive as defined below.

Further, the electron transport layer may comprise one or more additives. The additive may be an n-type dopant. The additive can be alkali metal, alkali metal compound, alkaline earth metal, alkaline earth metal compound, transition metal, transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. In another emdodiment, the n-type dopant can be one selected from a group consisting of Cs, K, Rb, Mg, Na, Ca, Sr, Eu and Yb. In an embodiment the alkali metal compound may be 8-Hydroxyquinolinolato-lithium (LiQ), Lithium tetra(1H-pyrazol-1-yl)borate or Lithium 2-(diphenylphosphoryl)phenolate. Suitable compounds for the ETM (which may be used in addition to the inventive compound represented by the general Formula (1) as defined above) are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1 970 371 A1 or WO 2013/079217 A1.

*Electron injection layer (EIL)*

An optional EIL, which may facilitates injection of electrons from the cathode, may be formed on the ETL,

preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

*Charge generation layer (CGL)*

The charge generation layer (CGL) may comprise a p- type charge generation layer (p-CGL) and an n-type charge generation layer (n-CGL). An interlayer may be arranged between the p-CGL and the -n-CGL.

Typically, the charge generation layer is a pn junction joining an n-type charge generation layer (electron generating layer) and a hole generating layer. The n-side of the pn junction generates electrons and injects them into the layer which is adjacent in the direction to the anode. Analogously, the p-side of the p-n junction generates holes and injects them into the layer which is adjacent in the direction to the cathode.

Charge generating layers are used in tandem and stacked devices, for example, in tandem or stacked OLEDs comprising, between two electrodes, two or more emission layers. In a tandem or stacked OLED comprising two emission layers, the n-type charge generation layer provides electrons for the first light emission layer arranged near the anode, while the hole generating layer provides holes to the second light emission layer arranged between the first emission layer and the cathode.

Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluore-7,7,8,8-tetracyanoquinodimethane (F4-TCNQ), derivatives of tetracyanoquinodimethane, radialene derivatives, iodine, FeCl3, FeF3, and SbCl5. Also, the host can be one selected from a group consisting of N,N'-di(naphthalen-i-yl)-N,N-diphenyl-benzidine (NPB), N,N'-diphenyl-N,N'-bis(3-methylphenyl)-1,1-biphenyl-4,4'-diamme (TPD) and N,N',N'-tetranaphthyl-benzidine (TNB). The p-type charge generation layer may consist of CNHAT.

The n-type charge generating layer may be the layer comprising the compound of Formula (I). The n-type charge generation layer can be layer of a neat n-type dopant, for example of a metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Li, Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds, hydroxyquinoline derivatives like tris(8-hydroxyquinoline)aluminum, benzazole derivatives, and silole derivatives.

The hole generating layer is arranged in direct contact to the n-type charge generation layer.

According to one aspect of the present invention, the organic semiconductor layer is arranged between the first and second emission layer and further comprises an n-type dopant.

According to one aspect of the present invention, the organic semiconductor layer is arranged between the first and second emission layer and further comprises a metal.

According to one aspect of the present invention, the organic semiconductor layer is arranged between the first and second emission layer and further comprises a metal selected from alkali, alkaline earth and rare earth metals.

According to one aspect of the present invention, the organic semiconductor layer comprising compound of Formula (1) is arranged between the first and second emission layer and a further organic semiconductor layer comprising compound of Formula (1) is arranged between the second emission layer and the cathode.

According to one aspect of the present invention, the organic semiconductor layer comprising compound of Formula (1) is arranged between the first and second emission layer and a further organic semiconductor layer comprising compound of Formula (1) is arranged between the second emission layer and the cathode; wherein the organic semiconductor layer comprising compound of Formula (1) further comprises an n-type dopant.

According to one aspect of the present invention, the organic semiconductor layer comprising compound of Formula (1) is arranged between the first and second emission layer and a further organic semiconductor layer comprising compound of Formula (1)is arranged between the second emission layer and the cathode; wherein the further organic semiconductor layer comprising compound of Formula (1) further comprises an n-type dopant.

According to one aspect of the present invention, the organic semiconductor layer comprising compound of Formula (1) is arranged between the first and second emission layer and a further organic semiconductor layer comprising

compound of Formula (1) is arranged between the second emission layer and the cathode; wherein the organic semiconductor layer comprising compound of Formula (1) further comprises an n-type dopant, and the further organic semiconductor layer comprising compound of Formula (1) further comprises an n-type dopant.

According to one aspect of the present invention, the organic semiconductor layer comprising compound of Formula (1) is arranged between the first and second emission layer and a further organic semiconductor layer comprising compound of Formula (1) is arranged between the second emission layer and the cathode; wherein the organic semiconductor layer comprising compound of Formula (1) further comprises a metal, and the further organic semiconductor layer comprising compound of Formula (1) further comprises an n-type dopant.

## Organic light-emitting diode

The organic electronic device according to the invention may be an organic light-emitting device.

According to one aspect of the present invention, there is provided an organic light-emitting diode (OLED) comprising: a substrate; an anode electrode formed on the substrate; a hole injection layer, a hole transport layer, an emission layer, an organic semiconductor layer comprising a compound of Formula (1) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an organic semiconductor layer comprising a compound of Formula (1) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an organic semiconductor layer comprising a compound of Formula (1), an electron injection layer, and a cathode electrode.

According to various embodiments of the present invention, there may be provided OLEDs layers arranged between the above mentioned layers, on the substrate or on the top electrode.

According to one aspect, the OLED can comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

The organic semiconductor layer according to the invention may be the electron transport layer, first electron transport layer, n-type charge generation layer and/or second electron transport layer.

For example, the OLED according to Fig. 2 may be formed by a process, wherein on a substrate (110), an anode (120), a hole injection layer (130), a hole transport layer (140), an electron blocking layer (145), an emission layer (150), a hole blocking layer (155), an electron transport layer (160), an electron injection layer (180) and the cathode electrode (190) are subsequently formed in that order.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

In one embodiment, the organic electronic device according to the invention comprising an organic semiconductor layer comprising a compound according to Formula (1) may further comprise a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb):

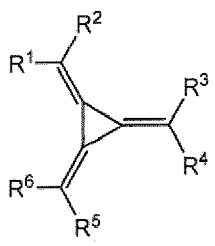
(XX)
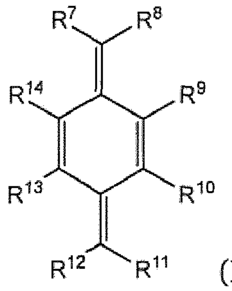
(XXIa)
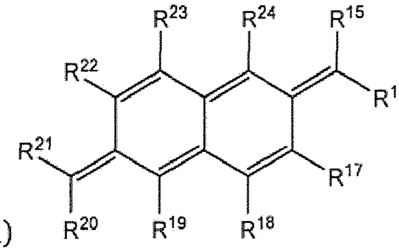
(XXIb),

wherein (as an exception different to the description above) $R^1$, $R^2$, $R^3$, R4, $R^5$, $R^6$, R7, $R^8$, $R^{11}$, $R^{12}$, $R^{15}$, $R^{16}$, $R^{20}$, $R^{21}$ are independently selected from above mentioned electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and above mentioned electron withdrawing groups.

According to one embodiment of the present invention, the organic semiconductor layer comprising compound of Formula (1) is adjacent to a layer comprising a compound of formula (XX), (XXIa) or (XXIb).

According to one embodiment of the present invention, the organic semiconductor layer comprising compound of Formula (1) is in direct contact to a layer comprising a compound of formula (XX), (XXIa) or (XXIb).

Display device

The present invention furthermore relates to a display device comprising an organic electronic device according to the present invention.

Method for manufacturing the organic electronic device

The organic electronic device according to the invention may be a light emitting device, or a photovoltaic cell, and preferably a light emitting device.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments of the present invention, there is provided a method using:

- a first deposition source to release the compound of Formula (1) according to the invention, and
- a second deposition source to release the metal, a metal salt or an alkali or alkaline earth metal complex; alternatively an organic alkali or alkaline earth metal complex; alternatively 8-hydroxyquinolinolato lithium;

the method comprising the steps of forming the organic semiconductor layer; whereby for an organic light-emitting diode (OLED):

- the organic semiconductor layer is formed by releasing the compound of Formula (1) according to the invention from the first deposition source and a metal, a metal salt or an alkali or alkaline earth metal complex; alternatively an organic alkali or alkaline earth metal complex; alternatively 8-hydroxyquinolinolato lithium, from the second deposition source.

According to various embodiments of the present invention, the method may further include forming on the anode electrode, an emission layer and at least one layer selected from the group consisting of forming a hole injection layer, forming a hole transport layer, or forming a hole blocking layer, between the anode electrode and the first electron transport layer.

According to various embodiments of the present invention, the method may further include the steps for forming an organic light-emitting diode (OLED), wherein

- on a substrate a first anode electrode is formed,
- on the first anode electrode an emission layer is formed,
- on the emission layer an electron transport layer stack is formed, optionally a hole blocking layer is formed on the emission layer and an organic semiconductor layer is formed,
- and finally a cathode electrode is formed,
- optional a hole injection layer, a hole transport layer, and a hole blocking layer, formed in that order between the first anode electrode and the emission layer,
- optional an electron injection layer is formed between the organic semiconductor layer and the cathode electrode.

According to various embodiments of the present invention, the method may further comprise forming an electron injection layer on the organic semiconductor layer. However, according to various embodiments of the OLED of the present invention, the OLED may not comprise an electron injection layer.

According to various embodiments, the OLED may have the following layer structure, wherein the layers having the following order:
anode, hole injection layer, first hole transport layer, second hole transport layer, emission layer, optional hole blocking layer, organic semiconductor layer comprising a compound of Formula (1) formula (according to the invention, optional electron injection layer, and cathode.

GENERAL DEFINITIONS

In the present specification, when a definition is not otherwise provided, an "alkyl group" may refer to an aliphatic hydrocarbon group. The alkyl group may refer to "a saturated alkyl group" without any double bond or triple bond. The term "alkyl" as used herein shall encompass linear as well as branched and cyclic alkyl. For example, $C_3$-alkyl may be selected from n-propyl and iso-propyl. Likewise, $C_4$-alkyl encompasses n-butyl, sec-butyl and t-butyl. Likewise, $C_6$-alkyl encompasses n-hexyl and cyclo-hexyl.

As used herein if not explicitly mentioned else, the asterisk symbol "*" represents a binding position at which the moiety labelled accordingly is bond to another moiety.

The subscribed number n in $C_{11}$ relates to the total number of carbon atoms in the respective alkyl, arylene, heteroarylene or aryl group.

The term "aryl" or "arylene" as used herein shall encompass phenyl ($C_6$-aryl), fused aromatics, such as naphthalene, anthracene, phenanthrene, tetracene etc.. Further encompassed are biphenyl and oligo- or polyphenyls, such as terphenyl, phenyl-substituted biphenyl, phenyl-substituted terphenyl (such as tetraphenyl benzole groups) etc.. "Arylene" respectively "heteroarylene", refers to groups to which two further moieties are attached. In the present specification, the term "aryl group" or "arylene group" may refer to a group comprising at least one hydrocarbon aromatic moiety, and all the elements of the hydrocarbon aromatic moiety may have p-orbitals which form conjugation, for example a phenyl group, a napthyl group, an anthracenyl group, a phenanthrenyl group, a pyrinyl group, a fluorenyl group and the like. Further encompoassed are spiro compounds in which two aromatic moieties are connected with each other via a spiro-atom, such as 9,9'-spirobi[9H-fluorene]yl. The aryl or arylene group may include a monocyclic or fused ring polycyclic (i.e., links sharing adjacent pairs of carbon atoms) functional group.

The term "heteroaryl" as used herein refers to aryl groups in which at least one carbon atom is substituted with a heteroatom. The term "heteroaryl" may refer to aromatic heterocycles with at least one heteroatom, and all the elements

of the hydrocarbon heteroaromatic moiety may have p-orbitals which form conjugation. The heteroatom may be selected from N, O, S, B, Si, P, Se, preferably from N, O and S. A heteroarylene ring may comprise at least 1 to 3 heteroatoms. Preferably, a heteroarylene ring may comprise at least 1 to 3 heteroatoms individually selected from N, S and/or O. Just as in case of "aryl"/"arylene", the term "heteroaryl" comprises, for example, spiro compounds in which two aromatic moieties are connected with each other, such as spiro[fluorene-9,9'-xanthene]. Further exemplary heteroaryl groups are diazine, triazine, dibenzofurane, dibenzothiofurane, acridine, benzoacridine, dibenzoacridine etc.

The term "alkenyl" as used herein refers to a group $-CR^1 = CR^2R^3$ comprising a carbon-carbon double bond.

The term "perhalogenated" as used herein refers to a hydrocarbyl group wherein all of the hydrogen atoms of the hydrocarbyl group are replaced by halogen (F, Cl, Br, I) atoms.

The term "alkoxy" as used herein refers to a structural fragment of the Formula -OR with R being hydrocarbyl, preferably alkyl or cycloalkyl.

The term "thioalkyl" as used herein refers to a structural fragment of the Formula -SR with R being hydrocarbyl, preferably alkyl or cycloalkyl.

The subscripted number n in $C_n$-heteroaryl merely refers to the number of carbon atoms excluding the number of heteroatoms. In this context, it is clear that a $C_3$ heteroarylene group is an aromatic compound comprising three carbon atoms, such as pyrazol, imidazole, oxazole, thiazole and the like.

The term "heteroaryl" as used herewith shall encompass pyridine, quinoline, benzoquinoline, quinazoline, benzoquinazoline, pyrimidine, pyrazine, triazine, benzimidazole, benzothiazole, benzo[4,5]thieno[3,2-d]pyrimidine, carbazole, xanthene, phenoxazine, benzoacridine, dibenzoacridine and the like.

In the present specification, the term single bond refers to a direct bond.

The term "fluorinated" as used herein refers to a hydrocarbon group in which at least one of the hydrogen atoms comprised in the hydrocarbon group is substituted by a fluorine atom. Fluorinated groups in which all of the hydrogen atoms thereof are substituted by fluorine atoms are referred to as perfluorinated groups and are particularly addressed by the term "fluorinated".

In terms of the invention, a group is "substituted with" another group if one of the hydrogen atoms comprised in this group is replaced by another group, wherein the other group is the substituent.

In accordance with the present disclosure, in a formula showing the following binding situation,

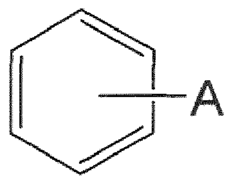

the group A may be bound to any suitable binding position. In a situation were it is shown that the bond of A crosses more than one ring

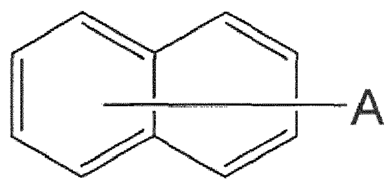

the group A may be bound to any suitable binding position of each ring crossed with the bond.

In terms of the invention, the expression "between" with respect to one layer being between two other layers does not exclude the presence of further layers which may be arranged between the one layer and one of the two other layers. In terms of the invention, the expression "in direct contact" with respect to two layers being in direct contact with each other means that no further layer is arranged between those two layers. One layer deposited on the top of another layer is deemed to be in direct contact with this layer.

The term "contacting sandwiched" refers to an arrangement of three layers whereby the layer in the middle is in direct contact with the two adjacent layers.

With respect to the inventive electron transport layer stack the compounds mentioned in the experimental part are most preferred.

A lighting device may be any of the devices used for illumination, irradiation, signaling, or projection. They are correspondingly classified as illuminating, irradiating, signaling, and projecting devices. A lighting device usually consists of a source of optical radiation, a device that transmits the radiant flux into space in the desired direction, and a housing

that joins the parts into a single device and protects the radiation source and light-transmitting system against damage and the effects of the surroundings.

According to another aspect, the organic electroluminescent device according to the present invention comprises two or three or more emission layers. An OLED comprising more than one emission layer is also described as a tandem OLED or stacked OLED.

The organic electroluminescent device (OLED) may be a bottom- or top-emission device. The organic electroluminescent device (OLED) may emit the light trough a transparent anode or through a transparent cathode.

Another aspect is directed to a device comprising at least one organic electroluminescent device (OLED).

A device comprising organic light-emitting diodes is for example a display or a lighting panel.

In the present invention, the following defined terms, these definitions shall be applied, unless a different definition is given in the claims or elsewhere in this specification.

In the context of the present specification the term "different" or "differs" in connection with the matrix material means that the matrix material differs in their structural Formula.

The terms "OLED" and "organic light-emitting diode" are simultaneously used and have the same meaning. The term "organic electroluminescent device" as used herein may comprise both organic light emitting diodes as well as organic light emitting transistors (OLETs).

As used herein, "weight percent", "wt.-%", "percent by weight", "% by weight", and variations thereof refer to a composition, component, substance or agent as the weight of that component, substance or agent of the respective electron transport layer divided by the total weight of the respective electron transport layer thereof and multiplied by 100. It is under-stood that the total weight percent amount of all components, substances and agents of the respective electron transport layer and electron injection layer are selected such that it does not exceed 100 wt.-%.

As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.

All numeric values are herein assumed to be modified by the term "about", whether or not explicitly indicated. As used herein, the term "about" refers to variation in the numerical quantity that can occur. Whether or not modified by the term "about" the claims include equivalents to the quantities.

It should be noted that, as used in this specification and the appended claims, the singular forms "a", "an", and "the" include plural referents unless the content clearly dictates otherwise.

The term "free of', "does not contain", "does not comprise" does not exclude impurities. Impurities have no technical effect with respect to the object achieved by the present invention.

In the context of the present specification the term "essentially non-emissive" or "non-emissive" means that the contribution of the compound or layer to the visible emission spectrum from the device is less than 10%, preferably less than 5 % relative to the visible emission spectrum. The visible emission spectrum is an emission spectrum with a wavelength of about ≥ 380 nm to about ≤ 780 nm.

Preferably, the organic semiconducting layer comprising the compound of Formula (1) is essentially non-emissive or non-emitting.

The operating voltage, also named U, is measured in Volt (V) at 10 milliAmpere per square centimeter (mA/cm2).

The candela per Ampere efficiency, also named cd/A efficiency is measured in candela per ampere at 10 milliAmpere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color. Efficiency values are compared at the same CIE-y.

The highest occupied molecular orbital, also named HOMO, and lowest unoccupied molecular orbital, also named LUMO, are measured in electron volt (eV).

The term "OLED", "organic light emitting diode", "organic light emitting device", "organic optoelectronic device" and "organic light-emitting diode" are simultaneously used and have the same meaning.

The term "life-span" and "lifetime" are simultaneously used and have the same meaning.

The anode and cathode may be described as anode electrode / cathode electrode or anode electrode / cathode electrode or anode electrode layer / cathode electrode layer.

Room temperature, also named ambient temperature, is 23° C.

Hereinafter, the embodiments are illustrated in more detail with reference to examples. However, the present disclosure is not limited to the following examples. Reference will now be made in detail to the exemplary aspects.

DESCRIPTION OF THE DRAWINGS

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

FIG. 1 is a schematic sectional view of an organic electronic device, according to an exemplary embodiment of the present invention;

FIG. 2 is a schematic sectional view of an organic light-emitting diode (OLED), according to an exemplary embodiment of the present invention;

FIG. 3 is a schematic sectional view of an OLED, according to an exemplary embodiment of the present invention.

FIG. 4 is a schematic sectional view of an OLED comprising a charge generation layer and two emission layers, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic electronic device 100, according to an exemplary embodiment of the present invention. The organic electronic device 100 includes a substrate no, an anode 120, a photoactive layer (PAL) 125, an organic semiconductor layer comprising a compound of formula (1) 160. The organic semiconductor layer comprising compound of formula (1) 160 is formed on the PAL 125. Onto the organic semiconductor layer 160, a cathode 190 is disposed.

FIG. 2 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an emission layer (EML) 150, an electron transport layer (ETL) 160. The electron transport layer (ETL) 160 is formed on the EML 150. Onto the electron transport layer (ETL) 160, an electron injection layer (EIL) 180 is disposed. The cathode 190 is disposed directly onto the electron injection layer (EIL) 180.

Instead of a single electron transport layer 160, optionally an electron transport layer stack (ETL) can be used.

Fig. 3 is a schematic sectional view of an OLED 100, according to another exemplary embodiment of the present invention. Fig. 3 differs from Fig. 2 in that the OLED 100 of Fig. 3 comprises an electron blocking layer (EBL) 145 and a hole blocking layer (HBL) 155.

Referring to Fig. 3, the OLED 100 includes a substrate 110, an anode 120, a hole injection layer (HIL) 130, a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode electrode 190.

Preferably, the organic semiconductor layer comprising a compound of Formula (1) may be an ETL.

Fig. 4 is a schematic sectional view of an OLED 100, according to another exemplary embodiment of the present invention. Fig. 4 differs from Fig. 3 in that the OLED 100 of Fig. 4 further comprises a charge generation layer (CGL) and a second emission layer (151).

Referring to Fig. 4, the OLED 100 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a second hole blocking layer (EBL) 156, a second electron transport layer (ETL) 161, a second electron injection layer (EIL) 181 and a cathode 190.

Preferably, the organic semiconductor layer comprising a compound of Formula (1) may be an n-type CGL.

Preferably, the organic semiconductor layer comprising a compound of Formula (1) may be the first ETL, n-type CGL and/or second ETL.

While not shown in Fig. 1, Fig. 2, Fig. 3 and Fig. 4, a sealing layer may further be formed on the cathode electrodes 190, in order to seal the OLEDs 100. In addition, various other modifications may be applied thereto.

Hereinafter, the embodiments are illustrated in more detail with reference to examples. However, the present disclosure is not limited to the following examples.

DETAILED DESCRIPTION

The invention is furthermore illustrated by the following examples which are illustrative only and non-binding.

EXPERIMENTAL PART

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

Rate onset temperature

The rate onset temperature ($T_{RO}$) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than 10-5 mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

Reduction potential

The reduction potential is determined by cyclic voltammetry with poteniostatic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard $Fc^+$/Fc redox couple, afforded finally the values reported above. All studied compounds as well as the reported comparative compounds showed well-defined reversible electrochemical behaviour.

Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment is determined by a semi-empirical molecular orbital method.

The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

Synthesis procedure

**5-(5-(3-(4,6-diphenyl-1,3,5-triazin-2-yl)phenyl)-3,6-diphenylpyrazin-2-yl)isoquinoline (Compound E3)**

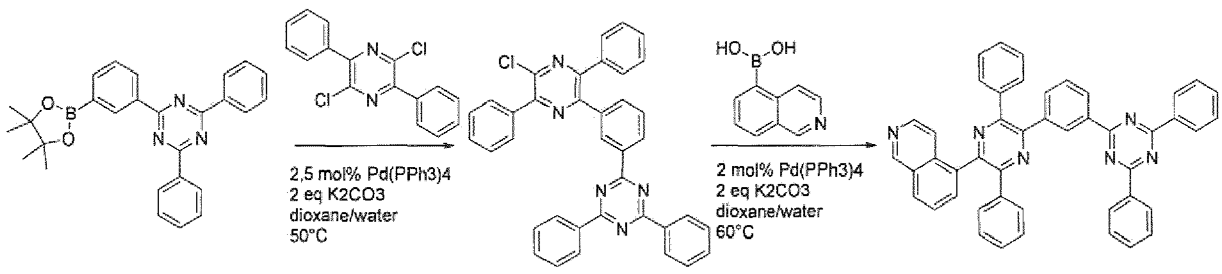


**1st Step. 2-(3-(5-chloro-3,6-diphenylpyrazin-2-yl)phenyl)-4,6-diphenyl-1,3,5-triazine**

3 Neck round-bottom flask was flushed with nitrogen and charged with 2,4-diphenyl-6-(3-(4,4,5,5-tetramethyl-1,3,2-dioxaborolan-2-yl)phenyl)-1,3,5-triazine (CAS 1269508-31-7) 1 eq (40,0 g), 2,5-dichloro-3,6-diphenylpyrazine (CAS 74134-61-5) 0,95 eq (26,3 g), tetrakis(triphenylphosphine)palladium(o) (CAS 14221-01-3) 0,025 eq (2,7 g), potassium carbonate (CAS 584-08-7) 2 eq (25,4 g). Deaerated mixture of 368 ml dioxane and 92 ml water was added. Reaction was running 2 days at 50°C under a nitrogen atmosphere. Yellow suspension was formed. Reaction was cooled down and precipitate was filtered and washed with dioxane, water and methanol. Raw product was dissolved in chlorobenzene and filtrated through a silica pad, solvent was evaporated at reduced pressure. Final purification was done by sublimation. Yield: 22,8 g (45%).

**2nd Step. 5-(5-(3-(4,6-diphenyl-1,3,5-triazin-2-yl)phenyl)-3,6-diphenylpyrazin-2-yl)isoquinoline**

3 Neck round-bottom flask was flushed with nitrogen and charged with 2-(3-(5-chloro-3,6-diphenylpyrazin-2-yl)phenyl)-4,6-diphenyl-1,3,5-triazine (no CAS) 1 eq (20,0 g), isoquinolin-5-ylboronic acid (CAS 371766-08-4) 1,5 eq (9,0 g), tetrakis(triphenylphosphine)palladium(0) (CAS 14221-01-3) 0,02 eq (0,8 g), potassium carbonate (CAS 584-08-7) 2 eq (9,6 g). Deaerated mixture of 200 ml dioxane and 40 ml water was added. Reaction was running for 22 hours at 60°C under a nitrogen atmosphere. After cooling down to room temperature brown suspension was formed. Raw solid material was filtered, dissolved in 600 ml chloroform and vigorously stirred for 3 hours with 30 ml 3% solution of sodium diethyldithiocarbamate. Organic phase was washed with water, dried over magnesium sulfate; solvent was removed on a rotary evaporator. Final purification was done by sublimation. Light orange powder. Yield: 20,1 g (77%). (ESI-APCI-MS: 667).

**2-(3-(3,6-diphenyl-5-(pyridin-3-yl)pyrazin-2-yl)phenyl)-4,6-diphenyl-1,3,5-triazine (Compound E4)**

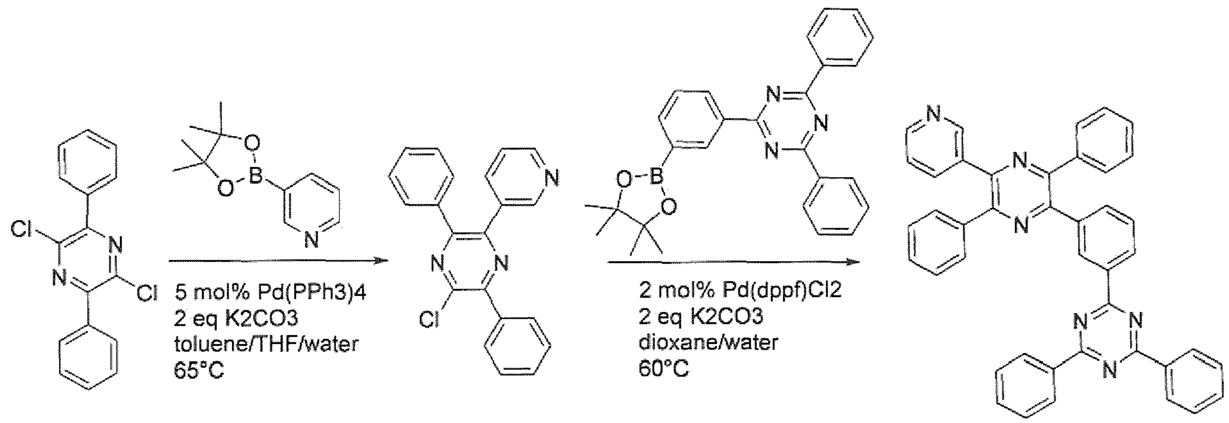


**1st Step. 2-chloro-3,6-diphenyl-5-(pyridin-3-yl)pyirazine**

5 Liter reactor was charged with 2,5-dichloro-3,6-diphenylpyrazine (CAS 74134-61-5) 1 eq (105,4 g), 3-(4,4,5,5-tetramethyl-1,3,2-dioxaborolan-2-yl)pyridine (CAS 329214-79-1) 1,05 eq (75,4 g), tetrakis(triphenylphosphine)palladium(0) (CAS 14221-01-3) 0,05 eq (20,2 g), potassium carbonate (CAS 584-08-7) 2 eq (96,7 g). Deaerated mixture of 1800 ml toluene/THF/water (1:1:1) was added. Reaction was running 3 days at 65°C under a nitrogen atmosphere. After reaction completion, solvent volume was reduced on rotary evaporator to 200 ml, extracted in DCM and washed with 200 ml brine. Organic phase was dried and chromatographed (1,5 kg silica gel, 5% ethyl acetate in chloroform) . Resulting product was stirred in 600 ml hexane/chloroform (5:1) at reflux for 2 h., filtered off and dried. Yield: 46,3 g (38%).

**2nd Step. 2-(3-(3,6-diphenyl-5-(pyridin-3-yl)pyrazin-2-yl)phenyl)-4,6-diphenyl-1,3,5-triazine**

3 Neck round-bottom flask was flushed with nitrogen and charged with 2-chloro-3,6-diphenyl-5-(pyridin-3-yl)pyrazine (no CAS) 1 eq (9,5 g), 2,4-diphenyl-6-(3-(4,4,5,5-tetramethyl-1,3,2-dioxaborolan-2-yl)phenyl)-1,3,5-triazine (CAS 1269508-31-7) 1 eq (12,0 g), [1,1'-Bis(diphenylphosphino)ferrocene]dichloropalladium(II) (CAS 72287-26-4) 0,02 eq (0,4 g), potassium carbonate (CAS 584-08-7) 2 eq (7,6 g). Deaerated mixture of 120 ml dioxane and 30 ml water was added. Reaction was running at 55°C under a nitrogen atmosphere overnight. Next day orange suspension was formed, reaction was cooled down; precipitate was filtered and washed with water and methanol. Product was dissolved in 800 ml chloroform and filtered through a silica pad. Final purification was done by sublimation. White solid. Yield: 12,1 g (71%) (ESI-MS: 617).

**2-(2-(3,6-diphenyl-5-(6-phenylpyridin-3-yl)pyrazin-2-yl)phenyl)-4, 6-diphenyl-1,3,5-triazine (Compound E6)**

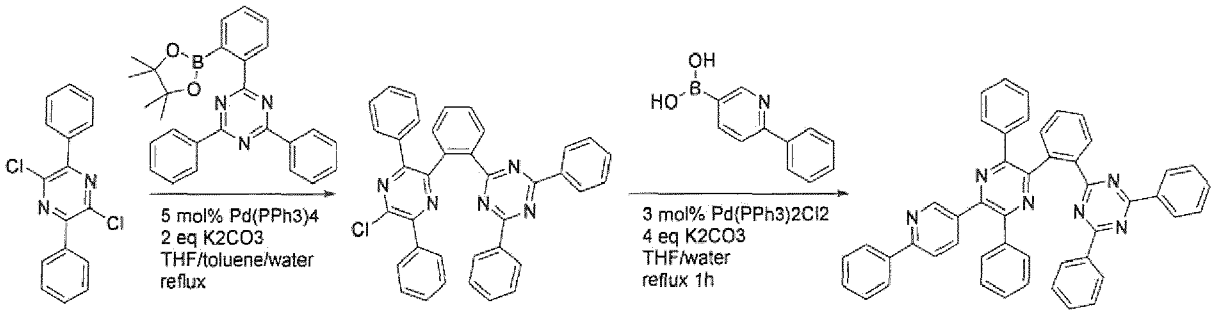


**1st Step. 2-(2-(5-chloro-3,6-diphenylpyrazin-2-yl)phenyl)-4,6-diphenyl-1,3,5-triazine**

3 Neck round-bottom flask was flushed with nitrogen and charged with 2,5-dichloro-3,6-diphenylpyrazine (CAS 74134-61-5) 1 eq (20,8 g), 2,4-diphenyl-6-(2-(4,4,5,5-tetramethyl-1,3,2-dioxaborolan-2-yl)phenyl)-1,3,5-triazine (CAS 1696425-27-0) 1 eq (30,0 g), tetrakis(triphenylphosphine)palladium(0) (CAS 14221-01-3) 0,05 eq (4,0 g), potassium carbonate (CAS 584-08-7) 2 eq (19,1 g). Deaerated mixture of THF/toluene/water (1:1:1) 540 ml was added. Reaction was running under an argon atmosphere for 44 hours at reflux. Then the reaction mixture was cooled down and extracted in DCM, wased with aq. K2CO3. Combined organic phases were dried and purified by column chromatography (2 kg silica gel, 50% DCM in petroleum ether). Yield: 16,1 g (41%).

**2nd Step. 2-(2-(3,6-diphenyl-5-(6-phenylpyridin-3-yl)pyrazin-2-yl)phenyl)-4,6-diphenyl-1,3,5-triazine**

3 Neck round-bottom flask was flushed with nitrogen and charged with 2-(2-(5-chloro-3,6-diphenylpyrazin-2-yl)phenyl)-4,6-diphenyl-1,3,5-triazine (no CAS) 1 eq (11,1 g), (6-phenylpyridin-3-yl)boronic acid (CAS 155079-10-0) 1,5 eq (5,8 g), bis(triphenylphosphine)-palladium-II-chloride (CAS 13965-03-2) 0,03 eq (0,4 g), potassium carbonate (CAS 584-08-7) 4 eq (io,6 g). Deaerated mixture of THF/water (3:1) 200 ml was added. Reaction was running under an argon atmosphere for 1 hour at reflux. Then the reaction mixture was cooled down and extracted from in DCM, washed with aq. $K2CO_3$. Combined organic phases were dried and purified by column chromatography (900 g silica gel, eluent DCM). Final purification was done by sublimation. White powder. Yield: 10,6 g (79%) (ESI-MS:693)

**2-(3-(3,6-diphenyl-5-(6-phenylpyridin-3-yl)pyrazin-2-yl)phenyl)-4,6-diphenyl-1,3,5-triazine (Compound E1)**

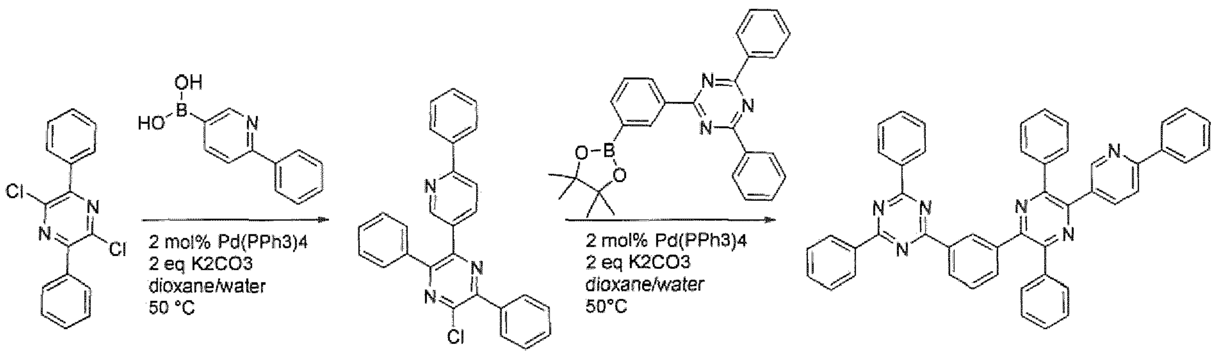


**1st Step. 2-chloro-3,6-diphenyl-5-(6-phenylpyridin-3-yl)pyrazine**

3 Neck round-bottom flask was flushed with nitrogen and charged with 2,5-dichloro-3,6-diphenylpyrazine (CAS 74134-61-5) 1eq (30,0 g), (6-phenylpyridin-3-yl)boronic acid (CAS 155079-10-0) 1,3 eq (25,8 g), tetrakis(triphenylphosphine)palladium(o) (CAS 14221-01-3) 0,02 eq (2,3 g), potassium carbonate (CAS 584-08-7) 2 eq (27,5 g). Deaerated mixture of 550 ml dioxane and 100 ml water was added. Reaction was running at 50°C under a nitrogen atmosphere for 2 days. Then the reaction mixture was cooled down, solvent was evaporated. Raw product was dissolved in 1 L DCM and washed with 200 ml water, dried over $MgSO_4$ and filtered through a silica pad. Solvent was evaporated under reduced pressure and the product was chromatographed (460 g silica gel, DCM/hexane 2:1). Yield: 14,3 g (35%).

**2nd Step. 2-(3-(3,6-diphenyl-5-(6-phenylpyridin-3-yl)pyrazin-2-yl)phenyl)-4,6-diphenyl-1,3,5-triazine**

3 Neck round-bottom flask was flushed with nitrogen and charged with 2,4-diphenyl-6-(3-(4,4,5,5-tetramethyl-1,3,2-dioxaborolan-2-yl)phenyl)-1,3,5-triazine (CAS 1269508-31-7) 1,1 eq (12,1 g), 2-chloro-3,6-diphenyl-5-(6-phenylpyridin-3-yl)pyrazine (no CAS) 1 eq (10,6 g), tetrakis(triphenylphosphine)palladium(o) (CAS 14221-01-3) 0,02 eq (0,6 g), potassium carbonate (CAS 584-08-7) 2 eq (7,0 g). Deaerated mixture of 400 ml dioxane and 30 ml water was added. Reaction was running at 50°C under a nitrogen atmosphere overnight. Next day gray suspension was formed, reaction was cooled down, precipitate was filtrated and washed with dioxane, water and methanol. Obtained raw product was dissolved in 1100 ml chloroform, filtrated through silica pad. Solvent was evaporated and product was recrystallized from THF. Final purification was done by sublimation. White powder. Yield: 8,7 g (52,8%)

**2-(3-(3,6-diphenyl-5-(3-(pyridin-4-yl)phenyl)pyrazin-2-yl)phenyl)-4,6-diphenyl-1,3,5-triazine (Compound E2)**

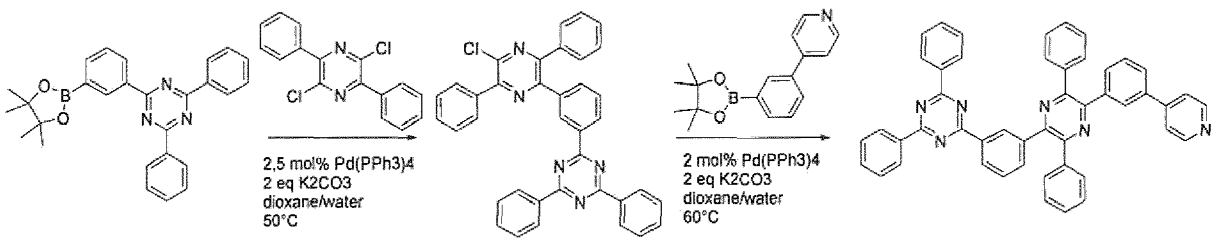


**1st Step. 2-(3-(5-chloro-3,6-diphenylpyrazin-2-yl)phenyl)-4,6-diphenyl-1,3,5-triazine**

3 Neck round-bottom flask was flushed with nitrogen and charged with 2,4-diphenyl-6-(3-(4,4,5,5-tetramethyl-1,3,2-dioxaborolan-2-yl)phenyl)-1,3,5-triazine (CAS 1269508-31-7) 1 eq (40,0 g), 2,5-dichloro-3,6-diphenylpyrazine (CAS 74134-61-5) 0,95 eq (26,3 g), tetrakis(triphenylphosphine)palladium(o) (CAS 14221-01-3) 0,025 eq (2,7 g), potassium carbonate (CAS 584-08-7) 2 eq (25,4 g). Deaerated mixture of 368 ml dioxane and 92 ml water was added. Reaction was running 2 days at 50°C under a nitrogen atmosphere. Yellow suspension was formed. Reaction was cooled down and precipitate was filtered and washed with dioxane, water and methanol. Raw product was dissolved in chlorobenzene and filtrated through a silica pad, solvent was evaporated at reduced pressure. Final purification was done by sublimation. Yield: 22,8 g (45%).

**2nd Step. 2-(3-(3,6-diphenyl-5-(3-(pyridin-4-yl)phenyl)pyraxin-2-yl)phenyl)-4,6-diphenyl-1,3 ,5- triazine**

3 Neck round-bottom flask was flushed with nitrogen and charged with 2-(3-(5-chloro-3,6-diphenylpyrazin-2-yl)phenyl)-4,6-diphenyl-1,3,5-triazine (no CAS) 1 eq (19,5 g), 4-(3-(4,4,5,5-tetramethyl-1,3,2-dioxaborolan-2-yl)phenyl)pyridine (CAS 1009033-83-3) 1,6 eq (15,3 g), tetrakis(triphenylphosphine)palladium(o) (CAS 14221-01-3) 0,02 eq (0,8 g), potassium carbonate (CAS 584-08-7) 2 eq (9,4 g). Deaerated mixture of 280 ml dioxane and 70 ml water was added. Reaction was running 2 days at 60°C under a nitrogen atmosphere. Brown suspension was formed. Reaction was cooled down and precipitate was filtrated and washed with dioxane, water and methanol. Raw product was dissolved in 370 ml toluene and filtered hot through silica pad. Final purification was done by sublimation. Pale yellow powder. Yield: 9,2 g (40%) (ESI-MS:693)

**3-(5-(3-(4,6-diphenyl-1,3,5-triazin-2-yl)phenyl)-3,6-diphenylpyrazin-2-yl)quinolone (Compound E5)**

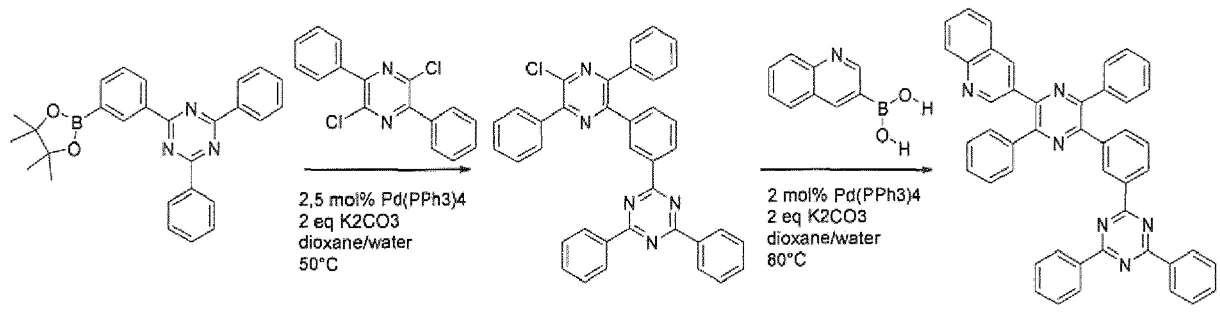


**1st Step. 2-(3-(5-chloro-3,6-diphenylpyrazin-2-yl)phenyl)-4,6-diphenyl-1,3,5-triazine**

3 Neck round-bottom flask was flushed with nitrogen and charged with 2,4-diphenyl-6-(3-(4,4,5,5-tetramethyl-1,3,2-dioxaborolan-2-yl)phenyl)-1,3,5-triazine (CAS 1269508-31-7) 1 eq (40,0 g), 2,5-dichloro-3,6-diphenylpyrazine (CAS 74134-61-5) 0,95 eq (26,3 g), tetrakis(triphenylphosphine)palladium(o) (CAS 14221-01-3) 0,025 eq (2,7 g), potassium carbonate (CAS 584-08-7) 2 eq (25,4 g). Deaerated mixture of 368 ml dioxane and 92 ml water was added. Reaction was running 2 days at 50°C under a nitrogen atmosphere. Yellow suspension was formed. Reaction was cooled down and precipitate was filtered and washed with dioxane, water and methanol. Raw product was dissolved in chlorobenzene and filtrated through a silica pad, solvent was evaporated at reduced pressure. Final purification was done by sublimation. Yield: 22,8 g (45%).

**2nd Step. 3-(5-(3-(4,6-diphenyl-1,3,5-triazin-2-yl)phenyl)-3,6-diphenylpyrazin-2-yl)quinoline**

3 Neck round-bottom flask was flushed with nitrogen and charged with 2-(3-(5-chloro-3,6-diphenylpyrazin-2-yl)phenyl)-4,6-diphenyl-1,3,5-triazine (no CAS) 1 eq (20,0 g), quinolin-3-ylboronic acid (CAS 191162-39-7) 1,4 eq (8,4 g), tetrakis(triphenylphosphine)palladium(o) (CAS 14221-01-3) 0,02 eq (0,8 g), potassium carbonate (CAS 584-08-7) 2 eq (9,6 g). Deaerated mixture of 140 ml dioxane and 40 ml water was added. Reaction was running 4 days at 80°C under a nitrogen atmosphere. Brown suspension was formed. Reaction was cooled down and precipitate was filtrated and washed with water and methanol. Raw product was dissolved in 850 ml chloroform and filtered through a silica pad and then recrystallized from toluene. Final purification was done by sublimation. White powder. Yield: 15,2 g (64,4%) (ESI-MS:667).

General procedure for fabrication of OLEDs

For the top emission OLED devices a substrate with dimensions of 150 mm x 150 mm x 0.7 mm was ultrasonically cleaned with a 2% aqueous solution of Deconex FPD 211 for 7 minutes and then with pure water for 20 minutes, and dried for 15 minutes in a spin rinse dryer. Subsequently, Ag was deposited as anode at a pressure of 10-5 to 10-7 mbar.

Then, HT-1 and D-1 were vacuum co-deposited on the anode to form a HIL. Then, HT-1 was vacuum deposited on the HIL to form an HTL. Then, HT-2 was vacuum deposited on the HTL to form an electron blocking layer (EBL).

Afterwards the emission layer was formed on the EBL by co-deposition of HOST-i and EMITTER-i.

The hole blocking layer (HBL) was formed on the emission layer by depositing compound ET-1.

Then, the compound of Formula (1) (selected from compounds E1 to E6), respectively the comparative compound (selected from compounds C1 to C2), was vacuum co-deposited with LiQ in the ratio 1:1 onto the hole blocking layer to form the electron transport layer.

Then, the electron injection layer is formed by depositing Yb.

A cap layer of HT-1 is formed on the cathode.

The details of the layer stack in the top emission OLED devices are given below. A slash "/" separates individual layers. Layer thicknesses are given in squared brackets [...], mixing ratios in wt% or vol% are given in round brackets (...):
Layer stack details used in the OLED device examples:
Ag [100nm] / HT-1:D-1 (wt% 92:8) [10 nm] / HT-1 [128 nm] / HT-2 [5 nm] / HOST-1:EMITTER-1 (vol% 97:3) [20 nm] / ET-1 [5 nm] / Compound of formula 1 or comparative compound [31 nm] / Yb [2 nm] / Ag:Mg (vol% 90:10) [13 nm] / HT-1 [75 nm].

Table 1: List of compounds used

| | IUPAC name | Reference |
|---|---|---|
| HT-1 | N-([1,1'-biphenyl]-4-yl)-9,9-dimethyl-N-(4-(9-phenyl-9H-carbazol-3-yl)phenyl)-9H-fluoren-2-amine [CAS 1242056-42-3] | US2016322581 |
| HT-2 | N-([1,1'-biphenyl]-4-yl)-9,9-diphenyl-N-(4-(triphenylsilyl) phenyl)-9H-fluoren-2-amine [CAS 1613079-70-1] | WO2014088047 |
| D-1 | 4,4',4"-((1E,1'E,1"E)-cyclopropane-1,2,3-triylidenetris (cyanomethanylylidene))tris(2,3,5,6-tetrafluorobenzonitrile) [CAS 1224447-88-4] | US2008265216 |
| HOST-1 | H09 (Fluorescent-blue host material) | Commercially available from Sun Fine Chemicals, Inc, S. Korea |
| EMITTER-1 | BD200 (Fluorescent-blue emitter material) | Commercially available from Sun Fine Chemicals, Inc, S. Korea |
| ET-1 | 2-(3'-(9,9-dimethyl-9H-fluoren-2-yi)-[1,1'-biphenyl]-3-yl)-4,6-diphenyl-1,3,5-triazine [CAS 1955543-57-3] | WO 2016105141 |
| LiQ | 8-Hydroxyquinolinolato-lithium [CAS 850918-68-2] | WO2013079217 |

Inventive compounds E1 to E6:

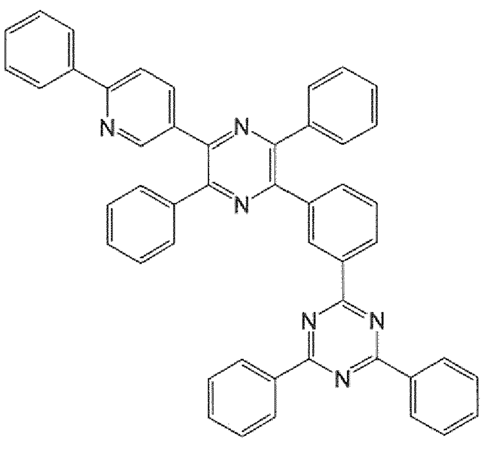

E1

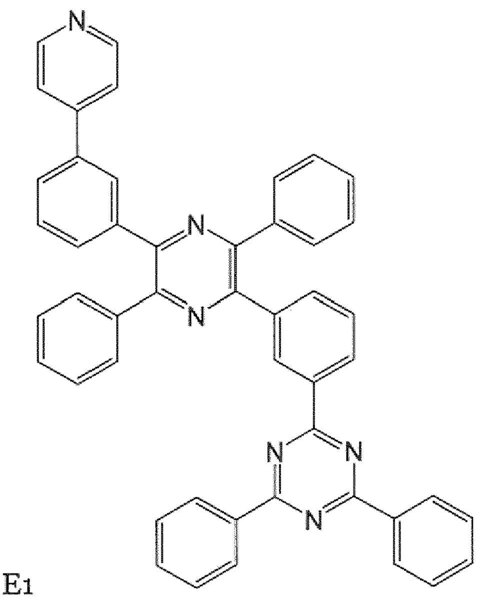

E2

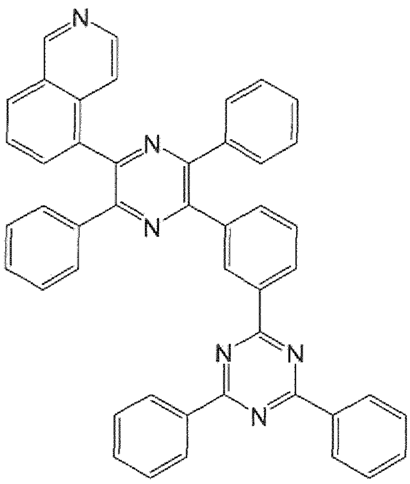
E3

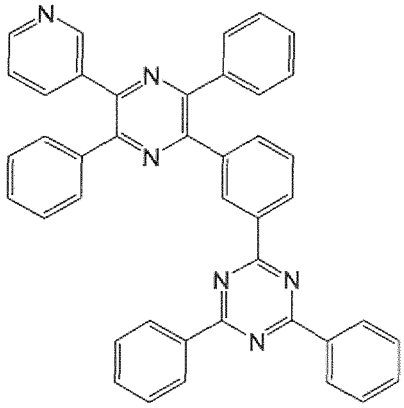
E4

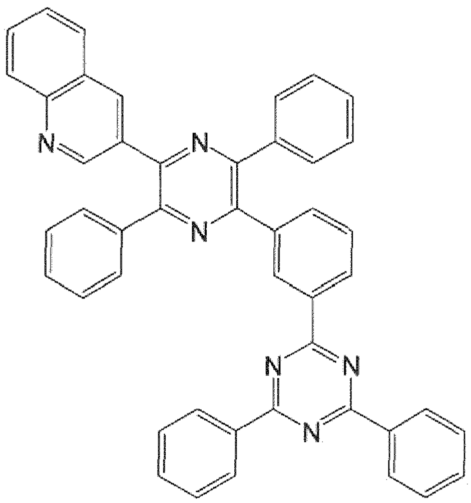
E5

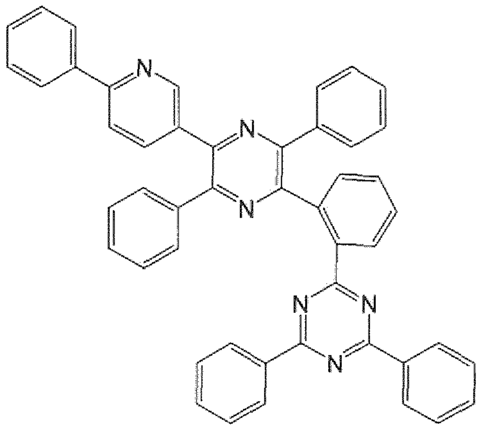
E6.

Comparative compounds C1 and C2

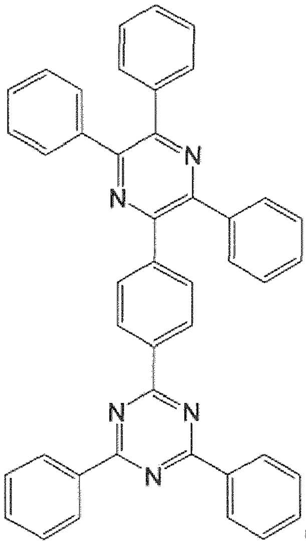

C1

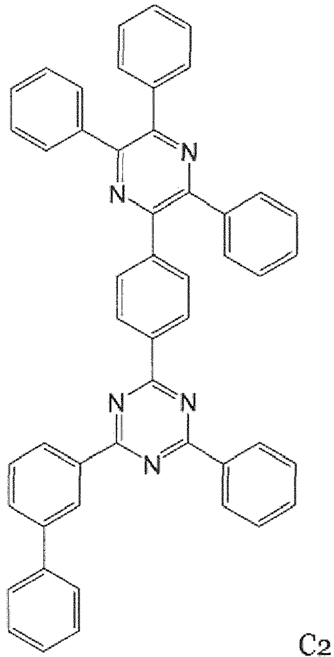

C2

Table 2: OLED device performance

| OLED devices | ETL | CIE 1931y | Voltage; 10 mA/cm$^2$ [V] | $C_{eff}$; 10 mA/cm$^2$ [cd/A] |
|---|---|---|---|---|
| Comparative-1 | $C_1$ | 0.042 | 3.75 | 6.9 |
| Comparative-2 | C2 | 0.042 | 3.8 | 6.8 |
| OLED-1 | E1 | 0.041 | 3.55 | 7.8 |
| OLED-2 | E2 | 0.039 | 3.53 | 7.6 |
| OLED-3 | E3 | 0.040 | 3.55 | 7.7 |
| OLED-4 | E4 | 0.039 | 3.55 | 7.5 |
| OLED-5 | E5 | 0.039 | 3.57 | 7.2 |
| OLED-6 | E6 | 0.040 | 3.59 | 7.6 |

As it can be seen from Table 2, he inventive devices OLED-1 to OLED-6 show improved driving voltage and improved efficiency in comparison to the comparative devices 1 and 2.

The features disclosed in the foregoing description and in the dependent claims may, both separately and in any combination thereof, be material for realizing the aspects of the disclosure made in the independent claims, in diverse forms thereof.

## Claims

**1.** Compound of formula (1)

$$Hy^2\text{-}L^l\text{-}Hy^1 \qquad (1)$$

wherein

- $Hy^1$ has the formula (2)

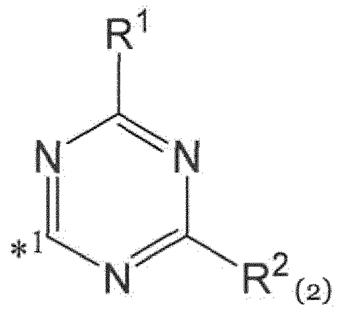

- $R^1$ and $R^2$ are independently selected from $C_6$ to $C_{60}$ aryl or $C_2$ to $C_{42}$ heteroaryl;

- $R^1$ and $R^2$ may independently be substituted with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
- each $C_6$ to $C_{12}$ aryl substituent on $R^1$ and/or $R^2$ and each $C_3$ to $C_{11}$ heteroaryl substituent on $R^1$ and/or $R^2$ may independently be substituted with $C_1$ to $C_4$ alkyl or halogen;

- $Hy^2$ has the formula (3)

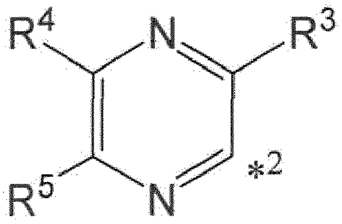
(3)

- wherein $R^3$ to $R^5$ are independently selected from $C_6$ to $C_{60}$ aryl or $C_2$ to $C_{42}$ heteroaryl;

- $R^3$ to $R^5$ may independently be substituted with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
- each $C_6$ to $C_{12}$ aryl substituent on $R^3$ to $R^5$ and each $C_3$ to $C_{11}$ heteroaryl substituent on $R^3$ to $R^5$ may independently be substituted with $C_1$ to $C_4$ alkyl or halogen;

- at least one of $R^3$ to $R^5$ has the formula (4)

$$*^3\text{-}L^2\text{-}Hy^3 \qquad (4)$$

- $*^3$-$L^2$-$Hy^3$ is bond at "*3" to the remaining structure of formula (3) ;
- $L^2$ is a single bond or phenylene;
- $Hy^3$ is $C_3$ to $C_{42}$ heteroaryl comprising at least one unsubstituted 6-membered N-containing heteroaromatic ring, wherein $L^2$ and $Hy^3$ are bonded via a single bond

- between $L^2$ and the 6-membered N-containing heteroaromatic ring; or

- between $L^2$ and a condensed ring system with two or three condensed rings, wherein one of the two or three rings is the 6-membered N-containing heteroaromatic ring;

- $L^1$ is selected from substituted or unsubstituted $C_6$ to $C_{60}$ arylene;

- $L^1$ may be substituted with one or more substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
- each $C_6$ to $C_{12}$ aryl substituent on L and each $C_3$ to $C_{11}$ heteroaryl substituent on L may independently be substituted with $C_1$ to $C_4$ alkyl or halogen;

- $L^1$ comprises at least one 6-membered aromatic ring, wherein the 6-membered aromatic ring has the formula (5) or (6)

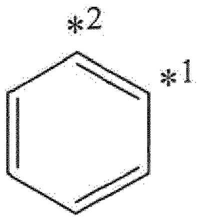
(5)

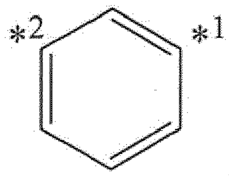
(6);

$Hy^1$ is bonded at "$*^1$" via a single bond to $L^1$; and
$Hy^2$ is bonded at "$*^2$" via a single bond to $L^2$;
wherein the following compounds are excluded:

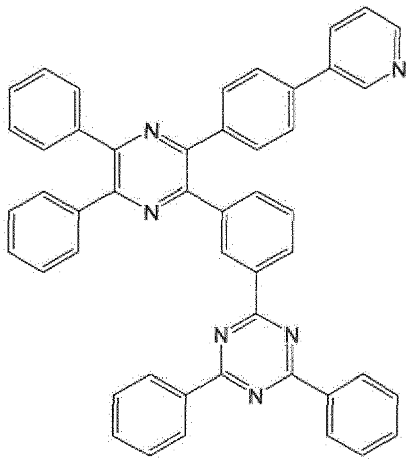
;

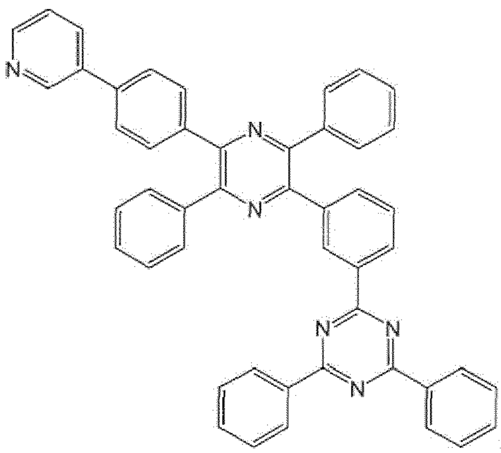
;

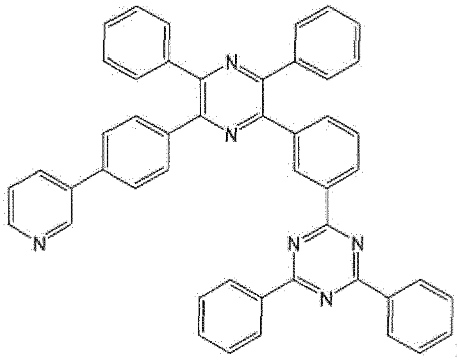

; and

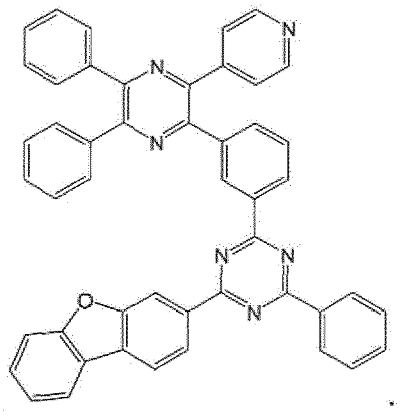

.

2. Compound according to claim 1, wherein $R^1$ and $R^2$ are independently selected from unsubstituted $C_6$ to $C_{60}$ aryl.

3. Compound according to claim 1 or 2, wherein $R^1$ and $R^2$ are independently selected from phenyl and biphenyl-yl.

4. Compound according to any of the preceding claims, wherein the structure according to formula (4) is selected from the following formulas (7) to (11)

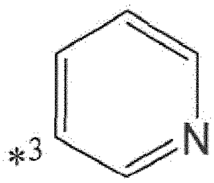
(7);
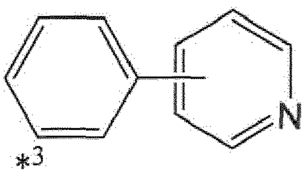
(8);
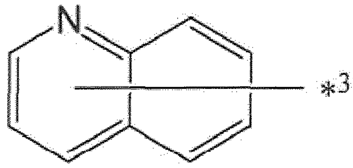
(9);
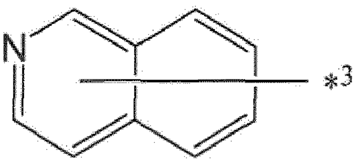
(10);
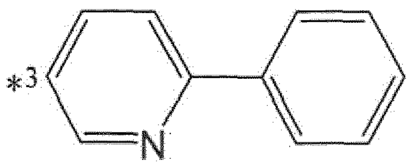
(11).

5. Compound according to any of the preceding claims, wherein $R^3$ and $R^5$ are independently selected from $C_6$ to $C_{60}$ aryl and $R^4$ has the formula (4) and the structure according to formula (4) is selected from the following formulas (7) to (11)

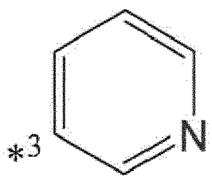
(7);
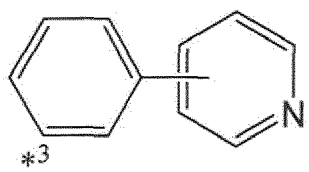
(8);
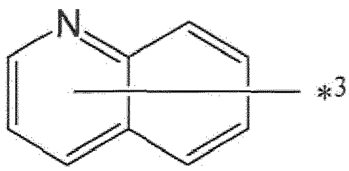
(9);
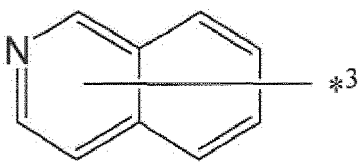
(10);
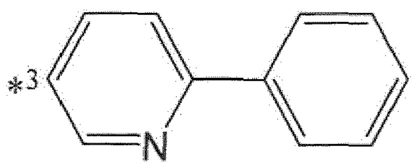
(11).

6. Compound according to any of the preceding claims, wherein the 6-membered aromatic ring comprised in $L^1$ has the formula (6)

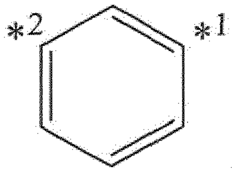

(6).

**7.** Compound according to any of the preceding claims, wherein $L^1$ has a formula selected from the following formulas (13) to (16)

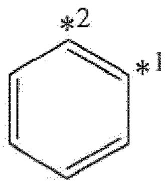

(13);

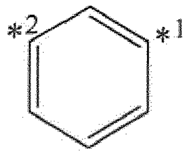

(14);

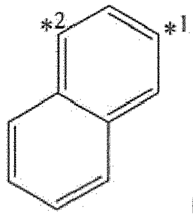

(15); and

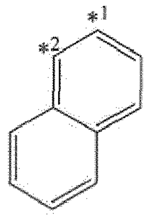

(16).

**8.** Compound according to any of the preceding claims, wherein $L^2$ is a single bond.

**9.** Compound according to any of the preceding claims, wherein the compound has a $|LUMO - LUMO+1| \leq 0.08$ eV.

**10.** Compound according to claim 9, wherein the compound meets the following requirement: $0.04 \leq |LUMO - LUMO+1| \leq 0.08$ eV.

**11.** Organic semiconducting material comprising the compound according to any of the preceding claims.

**12.** Organic semiconducting material according to claim 11, wherein the organic semiconducting material further comprises an n-dopant.

**13.** Organic electronic device comprising the organic semiconducting material according to claim 11 or 12.

**14.** Organic electronic device according to claim 13, wherein the organic electronic device is an organic light emitting diode.

**15.** Display device comprising the organic electronic device according to claim 14.

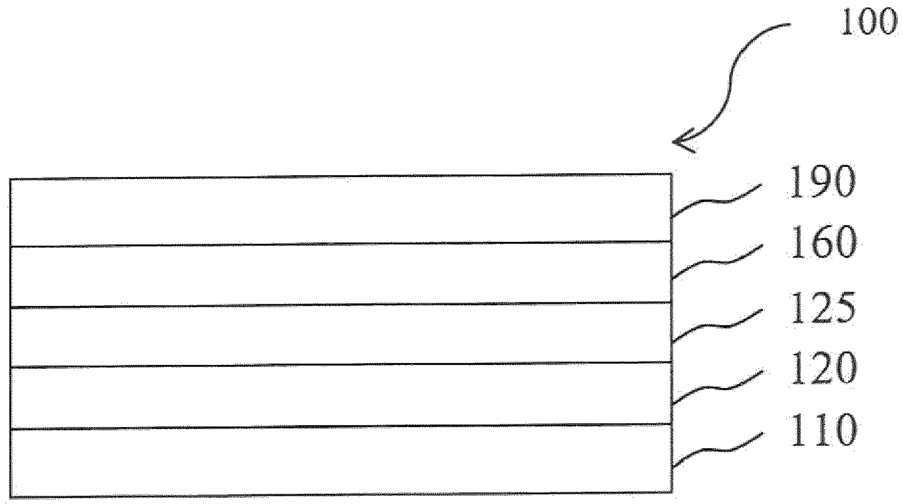


Fig.1

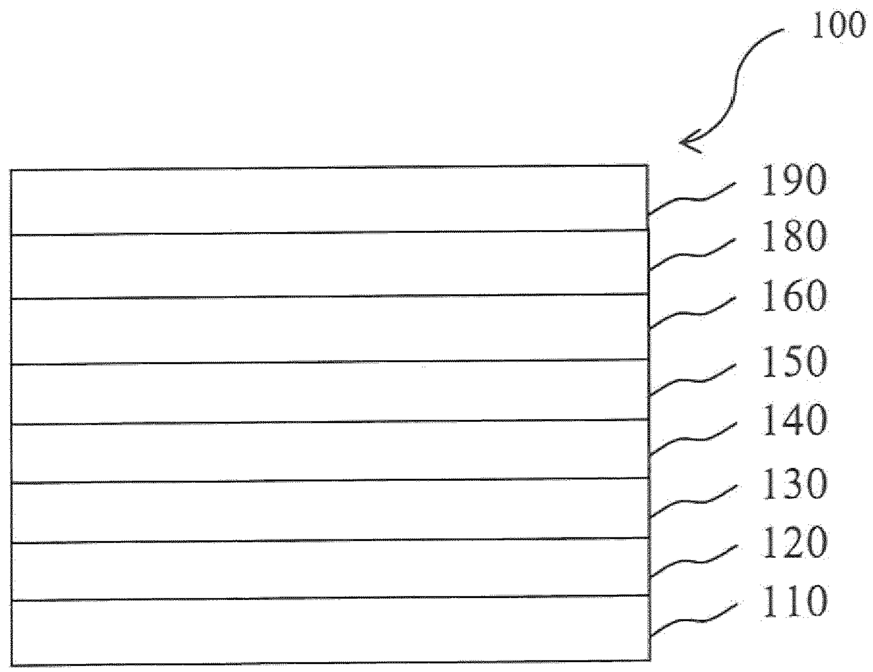


Fig.2

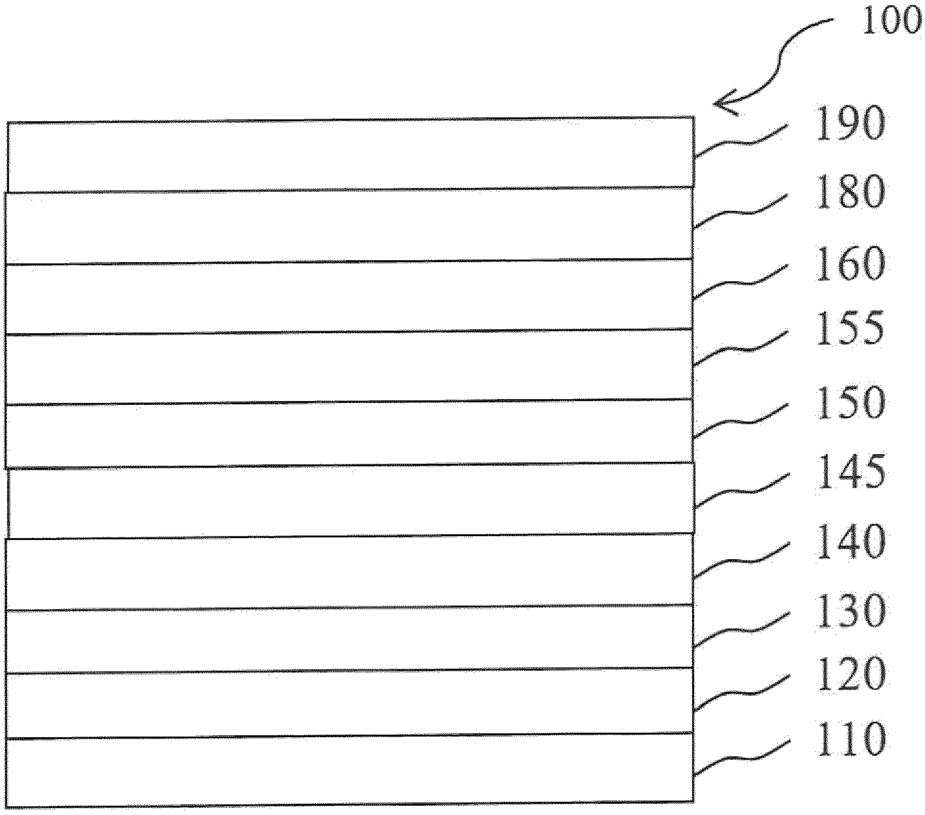


Fig.3

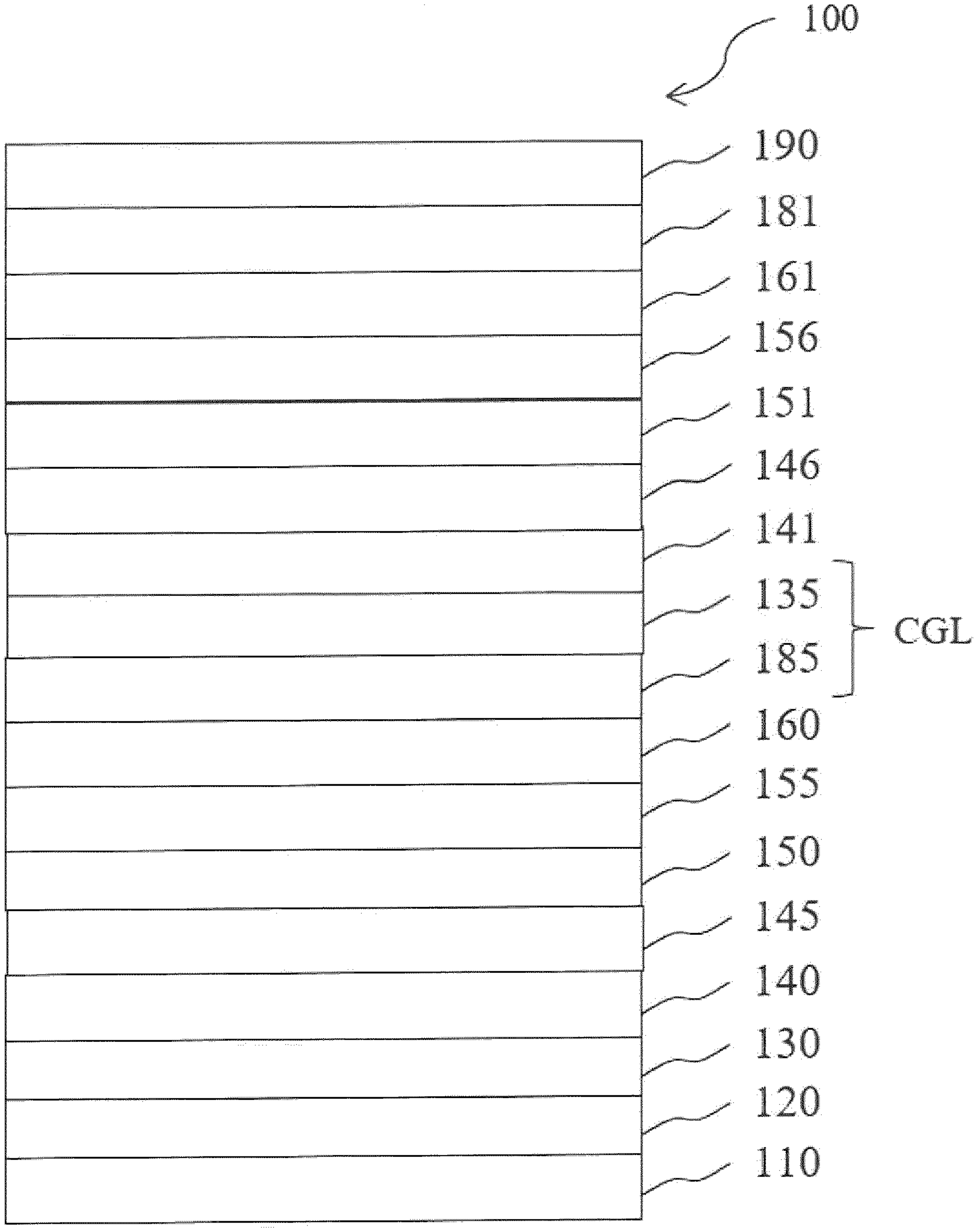
100
190
181
161
156
151
146
141
135
185
CGL
160
155
150
145
140
130
120
110

Fig.4

Europäisches Patentamt
European Patent Office
Office européen des brevets

# EUROPEAN SEARCH REPORT

Application Number
EP 22 18 9496

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/009206 A1 (NOVALED GMBH [DE]) 21 January 2021 (2021-01-21) * examples 130-131, 164-166 * ----- | 1-8, 11-15 | INV. C07D401/10 C09K11/06 |
| X | WO 2021/250277 A1 (NOVALED GMBH [DE]) 16 December 2021 (2021-12-16) * examples A9, C-6 * ----- | 1-15 | |
| X | EP 3 670 504 A1 (NOVALED GMBH [DE]) 24 June 2020 (2020-06-24) * examples * ----- | 1-15 | |
| A | WO 2020/120793 A1 (NOVALED GMBH [DE]) 18 June 2020 (2020-06-18) * examples * ----- | 1-15 | |
| X | EP 3 582 280 A1 (NOVALED GMBH [DE]) 18 December 2019 (2019-12-18) * examples * ----- | 1-15 | |

TECHNICAL FIELDS SEARCHED (IPC)

C07D
H05B
C09K

The present search report has been drawn up for all claims

1

| Place of search | Date of completion of the search | Examiner |
|---|---|---|
| Munich | 1 December 2022 | Fazzi, Raffaella |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 18 9496

This annex lists the patent family members relating to the patent documents cited in the above-mentioned European search report. The members are as contained in the European Patent Office EDP file on
The European Patent Office is in no way liable for these particulars which are merely given for the purpose of information.

01-12-2022

| Patent document cited in search report | Publication date | Patent family member(s) | Publication date |
|---|---|---|---|
| WO 2021009206 A1 | 21-01-2021 | CN 114222736 A | 22-03-2022 |
| | | EP 3766875 A1 | 20-01-2021 |
| | | EP 3999497 A1 | 25-05-2022 |
| | | KR 20220035181 A | 21-03-2022 |
| | | US 2022165958 A1 | 26-05-2022 |
| | | WO 2021009206 A1 | 21-01-2021 |
| WO 2021250277 A1 | 16-12-2021 | CN 115413374 A | 29-11-2022 |
| | | EP 3923364 A1 | 15-12-2021 |
| | | TW 202212301 A | 01-04-2022 |
| | | WO 2021250277 A1 | 16-12-2021 |
| EP 3670504 A1 | 24-06-2020 | CN 113195468 A | 30-07-2021 |
| | | EP 3670504 A1 | 24-06-2020 |
| | | EP 3898606 A1 | 27-10-2021 |
| | | KR 20210108976 A | 03-09-2021 |
| | | WO 2020126972 A1 | 25-06-2020 |
| WO 2020120793 A1 | 18-06-2020 | CN 113228325 A | 06-08-2021 |
| | | EP 3895227 A1 | 20-10-2021 |
| | | JP 2022511943 A | 01-02-2022 |
| | | KR 20210104100 A | 24-08-2021 |
| | | US 2022037595 A1 | 03-02-2022 |
| | | WO 2020120793 A1 | 18-06-2020 |
| EP 3582280 A1 | 18-12-2019 | CN 112368858 A | 12-02-2021 |
| | | EP 3582280 A1 | 18-12-2019 |
| | | EP 3807941 A1 | 21-04-2021 |
| | | JP 2021527327 A | 11-10-2021 |
| | | KR 20210021491 A | 26-02-2021 |
| | | TW 202000858 A | 01-01-2020 |
| | | US 2021198215 A1 | 01-07-2021 |
| | | WO 2019238858 A1 | 19-12-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005086251 A
- EP 1837926 B1
- WO 2007107306 A
- WO 2007107356 A
- EP 2722908 A1
- EP 1970371 A1
- WO 2013079217 A1
- US 2016322581 A
- WO 2014088047 A
- US 2008265216 A
- WO 2016105141 A
- WO 2013079217 A


### Non-patent literature cited in the description


- **YASUHIKO SHIROTA ; HIROSHI KAGEYAMA.** *Chem. Rev.,* 2007, vol. 107, 953-1010
- **KURT J.** *Lesker Company, www.lesker.com*
- *CreaPhys GmbH, http://www.creaphys.com*
- *CHEMICAL ABSTRACTS,* 1269508-31-7
- *CHEMICAL ABSTRACTS,* 74134-61-5
- *CHEMICAL ABSTRACTS,* 14221-01-3
- *CHEMICAL ABSTRACTS,* 584-08-7
- *CHEMICAL ABSTRACTS,* 371766-08-4
- *CHEMICAL ABSTRACTS,* 329214-79-1
- *CHEMICAL ABSTRACTS,* 72287-26-4
- *CHEMICAL ABSTRACTS,* 1696425-27-0
- *CHEMICAL ABSTRACTS,* 155079-10-0
- *CHEMICAL ABSTRACTS,* 13965-03-2
- *CHEMICAL ABSTRACTS,* 1009033-83-3
- *CHEMICAL ABSTRACTS,* 191162-39-7
- *CHEMICAL ABSTRACTS,* 1242056-42-3
- *CHEMICAL ABSTRACTS,* 1613079-70-1
- *CHEMICAL ABSTRACTS,* 1224447-88-4
- *CHEMICAL ABSTRACTS,* 1955543-57-3
- *CHEMICAL ABSTRACTS,* 850918-68-2